(19) Europäisches Patentamt
European Patent Office
Office européen des brevets

(11) Publication number: **0 139 393 B1**

# (12) EUROPEAN PATENT SPECIFICATION

(45) Date of publication of patent specification: **15.05.91**

(51) Int. Cl.5: **C07D 471/04**, A61K 31/505, //(C07D471/04,239:00,221:00)

(21) Application number: **84305684.7**

(22) Date of filing: **21.08.84**

## (54) Pyrimido(4,5-g)quinolines, process for their preparation, and pharmaceutical compositions containing them.

(30) Priority: **26.09.83 US 535503**
**26.09.83 US 535474**
**26.09.83 US 535519**
**30.01.84 US 575126**
**02.05.84 US 606091**

(43) Date of publication of application:
**02.05.85 Bulletin 85/18**

(45) Publication of the grant of the patent:
**15.05.91 Bulletin 91/20**

(84) Designated Contracting States:
**AT BE CH DE FR IT LI LU NL SE**

(56) References cited:
**EP-A- 0 013 787**

**THE JOURNAL OF ORGANIC CHEMISTRY, vol. 47, no. 13, 18th June 1982, pages 2673-2675, Columbus, Ohio, US; D.L. BOGER et al.: "Thermal cycloaddition of 1,3,5-triazine with enamines: Regiospecific pyrimidine annulation"**

(73) Proprietor: **ELI LILLY AND COMPANY**
**307, East McCarty Street**
**Indianapolis Indiana 46285(US)**

(72) Inventor: **Nichols, Cynthia Lynn**
**6106 Norwaldo Avenue**
**Indianapolis Indiana 46220(US)**
Inventor: **Kornfeld, Edmund Carl**
**5159 East 76th Court**
**Indianapolis Indiana 46250(US)**
Inventor: **Foreman, Mark Mortensen**
**7626 Dartmouth Road**
**Indianapolis Indiana 46260(US)**
Inventor: **Schaus, John Mehnert**
**5427 North Delaware Street**
**Indianapolis Indiana 46220(US)**
Inventor: **Huser, Diane Lynn**
**5620 North Broadway**
**Indianapolis Indiana 46220(US)**
Inventor: **Booher, Richard Nolan**
**6886 Hillcrest Court**
**Indianapolis Indiana 46227(US)**
Inventor: **Wong, David Taiwai**
**1640 Ridge Hill Lane**
**Indianapolis Indiana 46217(US)**

EP 0 139 393 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(74) Representative: Tapping, Kenneth George et al
Lilly Industries Limited Patent Department
Erl Wood Manor
Windlesham Surrey, GU20 6PH(GB)

## Description

This invention concerns a class of novel pyrimido[4,5-g]quinoline derivatives, which have been discovered to be effective D-1 and D-2 dopamine agonists.

The concept that various body tissues contain two dopamine receptors has only recently received general acceptance. These receptors have been designated as the D-1 and D-2 receptors. Several D-2 dopamine receptor agonists are known, including lergotrile and pergolide, both ergolines, and LY141865 (U.S. patent 4,198,415) an ergoline partial structure. These D-2 agonists have been found useful in treating Parkinson's disease as well as conditions in which there is an excess of circulating prolactin such as galactorrhea. LY141865 [trans-(±)-5-n-propyl-4,4a,5,6,7,8,8a,9-octahydro-1H(and 2H)-pyrazolo[3,4-g]-quinoline] has also been found to reduce blood pressure in mammals without the occurrence of postural hypotension. This anti-hypertensive activity is stated to be present in only one of the stereoisomers of the trans-(±) racemate, the trans-(-)isomer, named as 4aR,8aR-5-n-propyl-4,4a,5,6,7,8,8a,9-octahydro-1H(and 2H)-pyrazolo[3,4-g]quinoline.

This invention provides trans-(±)-2,4,6-permissibly substituted-5,5a,6,7,8,9,9a,10-octahydropyrimido[4,5-g]quinolines represented by the formula

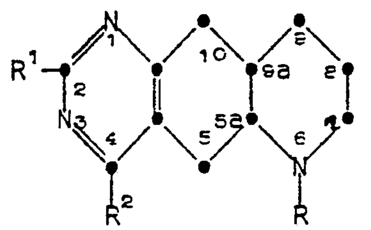

I

wherein:
R is $C_1$-$C_3$ alkyl or allyl;
$R^2$ is H, $CH_3$, Cl or Br;
$R^1$ is $NH_2$, $NHR^3$ or $NR^4R^5$;
wherein $R^3$ is methyl, ethyl, n-propyl or $R^6$-CO where $R^6$ is $C_1$-$C_3$ alkyl or

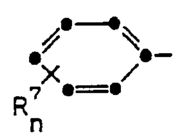

where $R^7$ is independently H, Cl, F, Br, $CH_3$, $C_2H_5$, $CH_3O$, $C_2H_5O$ or $CF_3$; and n is 0, 1 or 2;
wherein $R^4$ and $R^5$ are independently methyl, ethyl or n-propyl; or
a pharmaceutically-acceptable acid addition salt thereof.

Various terms used in this application are defined as follows. The term "$C_1$-$C_4$ alkyl" includes methyl, ethyl, n-propyl, isopropyl, n-butyl, sec-butyl, and tert-butyl. The term "$C_1$-$C_3$ alkyl" is included within the $C_1$-$C_4$ alkyl term. The term "$C_1$-$C_3$ alkoxy" includes methoxy, ethoxy, n-propoxy and isopropoxy.

Pharmaceutically-acceptable acid addition salts of the compounds of formula I include salts derived from non-toxic inorganic acids such as hydrochloric acid, nitric acid, phosphoric acid, sulfuric acid, hydrobromic acid, hydriodic acid, phosphorous acid and the like, as well as salts derived from non-toxic organic acids such as aliphatic mono and dicarboxylic acids, phenyl-substituted alkanoic acids, hydroxy alkanoic and alkandioic acids, aromatic acids, aliphatic and aromatic sulfonic acids. Such pharmaceutically-acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycollate, malate, tartrate, methanesulfonate, propanesulfonate, naphthalene-1-sulfonate, naphthalene-2-sulfonate and the like salts.

Compounds of formula I have two asymmetric carbons (optical centers) at 5a and 9a and can thus exist as four stereoisomers occurring as two racemic pairs, ordinarily designated as the trans-(±) racemate and the cis-(±) racemate. The trans racemate is composed of the trans-(-)-stereoisomer (5aR,9aR stereoisomer) represented by II below and the trans-(+)-(5aS,9aS stereoisomer) represented by IIa.

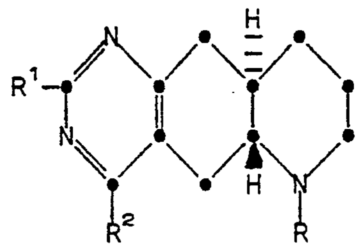

II

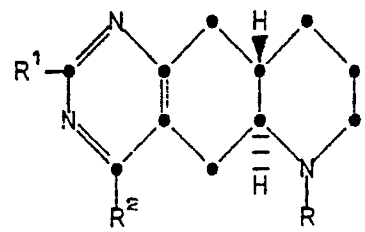

IIa

wherein R, $R^1$ and $R^2$ have their previously assigned meanings. These trans-(-)-(5aR,9aR) stereoisomers represented by formula II are active dopamine D-2 agonists. Compounds according to formula II thus form a second aspect of this invention. The trans-(+)-(5aS,9aS) stereoisomers of formula IIa are active dopamine D-1 agonists. Compounds according to formula IIa thus form a third aspect of this invention. Because the trans-(+) D-1 agonists are less active on a dosage basis than the trans-(-) D-2 agonists, the trans-(±)-racemates (II + IIa) are chiefly useful for their content of the active trans-(-)-stereoisomer.

A preferred group of compounds are those of formulae I, II and IIa in which R is n-propyl. Another preferred group of compounds are those of formulae I, II and IIa in which $R^1$ is $NH_2$ and/or $R^2$ is H.

Compounds of formulae I, II and IIa include, for example:
5aR,9aR-2-diethylamino-6-ethyl-5,5a,6,7, 8,9,9a,10-octahydropyrimido[4,5-g]quinoline sulfate,
trans-(±)-2-methylethylamino-4-methyl-6-n-propyl-5,5a,6,7,8,9,9a,10-octahydropyrimido[4,5-g]quinoline monohydrophosphate,
trans-(±)-2-n-propylamino-6-n-propyl-5,5a,6,7,8,9,9a,10-octahydropyrimido[4,5-g]quinoline maleate,
5aR,9aR-2-amino-4-chloro-6-ethyl-5,5a,6,7,8,9,9a,10-octahydropyrimido[4,5-g]quinoline succinate,
trans-(±)-2-amino-4-bromo-6-allyl-5,5a,6,7,8,9,9a,10-octahydropyrimido[4,5-g]quinoline phthalate,
trans-(±)-2-n-propylamino-6-n-propyl-5,5a,6,7,8,9,9a,10-octahydropyrimido[4,5-g]quinoline mesylate,
trans-(±)-2-amino-6-methyl-5,5a,6,7,8,9,9a,10-octahydropyrimido[4,5-g]quinoline hydrochloride,
5aR,9aR-2-amino-4,6-dimethyl-5,5a,6,7,8,9,9a,10-octahydropyrimido[4,5-g]quinoline tosylate,
5aR,9aR-2-acetamido-6-methyl-6-n-propyl-5,5a,6,7,8,9,9a,10-octahydropyrimido[4,5-g]quinoline dihydrobromide,
5aR,9aR-2-benzamido-6-ethyl-5,5a,6,7,8,9,9a,10-octahydropyrimido[4,5-g]quinoline hexan-1,6-dioate,
trans-(±)-2-n-propylamino-6-isopropyl-5,5a,6,7,8,9,9a,10-octahydropyrimido[4,5-g]quinoline dinitrobenzoate,
5aS,9aS-2-methylamino-6-methyl-5,5a,6,7,8,9,9a,10-octahydropyrimido[4,5-g]quinoline maleate,
5aS,9aS-2-amino-6-ethyl-5,5a,6,7,8,9,9a,10-octahydropyrimido[4,5-g]quinoline sulfate,
5aS, 9aS-2-dimethylamino-4-methyl-6-n-propyl-5,5a,6,7,8,9,9a,10-octahydropyrimido[4,5-g]quinoline succinate,
5aS,9aS-2-ethylamino-4-chloro-6-ethyl-5,5a,6,7,8,9,9a,10-octahydropyrimido[4,5-g]quinoline mesylate,
5aS, 9aS-2-allylamino-6-n-propyl-5,5a,6,7,8,9,9a,10-octahydropyrimido[4,5-g]quinoline phosphate,
5aS,9aS-2-methylethylamino-6-allyl-5,5a,6,7,8,9,9a,10-octahydropyrimido[4,5-g]quinoline citrate,
5aS,9aS-2-methylamino-6-allyl-4-methyl-5,5a,6,7,8,9,9a,10-octahydropyrimido[4,5-g]quinoline lactate,
5aS,9aS-2-amino-6-allyl-4-bromo-5,5a,6,7,8,9,9a,10-octahydropyrimido[4,5-g]quinoline sulfate,
5aS,9aS-2-dimethylamino-6-n-propyl-5,5a,6,7,8,9,9a,10-octahydropyrimido[4,5-g]quinoline maleate,
5aS,9aS-2-dimethylamino-6-ethyl-5,5a,6,7,8,9,9a,10-octahydropyrimido[4,5-g]quinoline maleate, and the like.

The compounds of formulae I, II and IIa are prepared by:

(a) condensing a compound of the formula

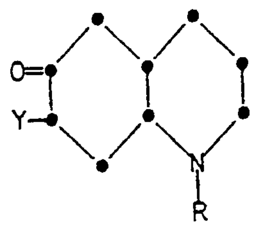

V

wherein R is as defined as above;
Y is $CH_3CO$, or $(R^{10})_2NCH=$, wherein each $R^{10}$ is independently $C_1$-$C_3$ alkyl, or one $R^{10}$ is H and the other is $C_1$-$C_3$ alkyl;
with a compound or its salt of the formula

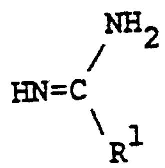

VI

wherein $R^1$ is defined as above to provide a compound of formula I; or

(b) alkylating a compound of the formula

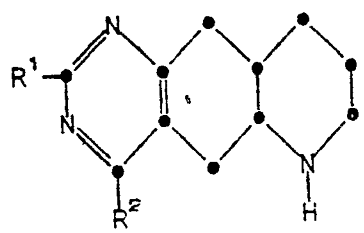

Ia

wherein $R^1$ and $R^2$ are defined as above to provide the compounds of formula I; or
(c) halogenating a compound of the formula

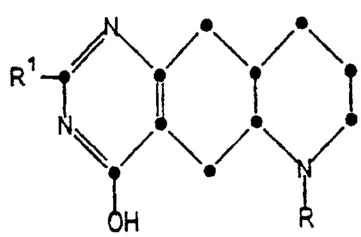

If

wherein R and $R^1$ are defined as above to provide the compounds of formula I where $R^2$ is Cl or Br; or
(d) acylating a compound of formula I wherein $R^1$ is $NH_2$ to provide a compound of formula I in which $R^1$ is $NHR^3$ where $R^3$ is $R^6$-CO; and
(e) optionally followed by salifying to form the pharmaceutically-acceptable acid addition salt of the product of formula I and/or resolving a racemic product to form the optically active product of formula I.

The compounds of formula I, II or IIa are used as drugs either as the free base or as a pharmaceutically-acceptable acid addition salt thereof.

Compounds represented by formulae I and II are dopamine D-2 agonists substantially devoid of other agonist or antagonist (blocking) activities. As D-2 dopamine agonists, the compounds are useful in treating Parkinson's Syndrome, in treating sexual dysfunction, as anti-depressants or as anti-anxiety agents, in lowering blood pressure in hypertensive mammals and in inhibiting prolactin secretion. Thus, the compounds of formulae I and II are useful in the treatment of hypertension, of depression, of anxiety, of Parkinson's disease, of sexual dysfunction and of disease states characterized by an excess of prolactin

secretion such as galactorrhea and inappropriate lactation.

Compounds represented by formula IIa are dopamine D-1 agonists. Dopamine D-1 receptors, when stimulated by a D-1 agonist, are characterized by an increased cyclic AMP efflux. This effect is inhibited by D-2 agonists. Stoof and Kebabian discuss these D-1 agonist effects in papers appearing in Nature, 294, 266 (1981) and Brain Res., 250, 263 (1982). The compounds of formula IIa are potentially useful as renal vasodilators and thus useful in the treatment of hypertension.

A still further embodiment of this invention is the provision of pharmaceutical formulations for administering drugs of formulae I, II and IIa in the treatment methods outlined above. Encompassed within this invention are pharmaceutical formulations which comprise as an active ingredient a compound of formula I, II or IIa or a pharmaceutically-acceptable salt thereof, associated with one or more pharmaceutically-acceptable carriers or diluents therefor.

Intermediates useful in preparing the compounds of formula I, II or IIa are represented by the formulae

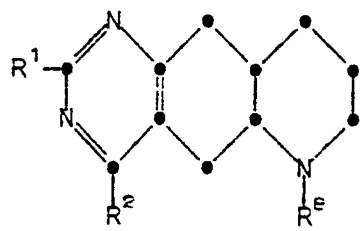

III

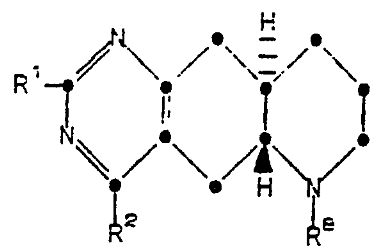

IV

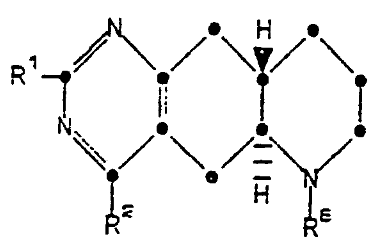

IVa

wherein $R^8$ is H, CN or ($C_1$-$C_3$ alkoxy)-CO, and $R^1$ and $R^2$ have the same meanings as in formula I above, or an acid addition salt thereof.

Another class of intermediates useful in preparing the compounds of formula I, II or IIa are represented by the formula

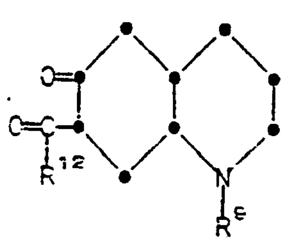

Va

where $R^9$ is H, CN, $C_1$-$C_3$ alkyl or allyl; and $R^{12}$ is methyl, or $C_1$-$C_3$ alkoxy

Compounds of formula I, II or IIa wherein $R^2$ is H can be prepared as shown in the following reaction scheme:

## Synthetic Route 1

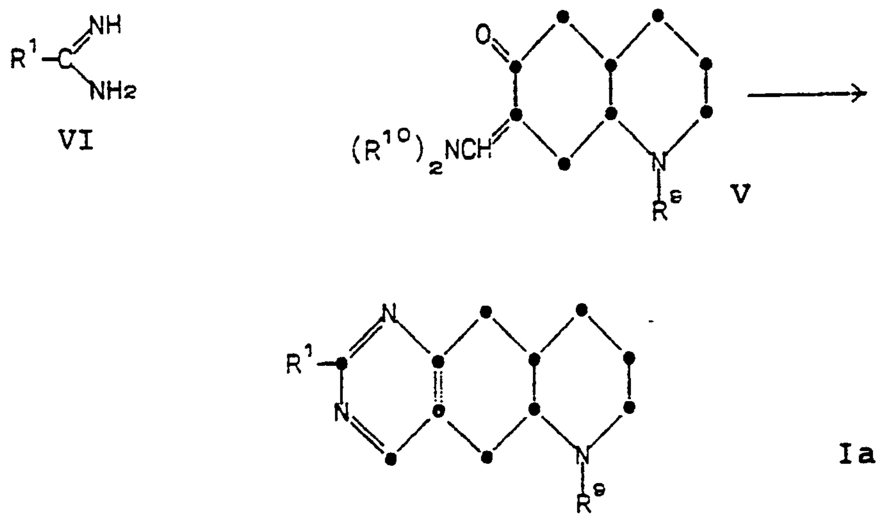

VI

V

Ia

wherein $R^1$, $R^9$ and $R^{10}$ have their previous meanings.

The procedure is equally applicable to the synthesis of the trans-(-)-stereoisomer (Ib) or of the trans-(+)-stereoisomer (Ic),

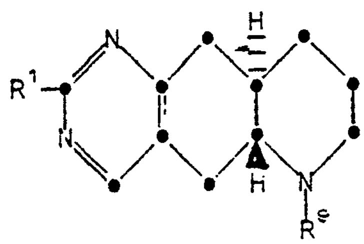

Ib

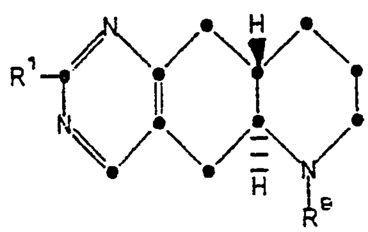

Ic

wherein $R^1$ and $R^9$ have the same significance as before. Suitable solvents are polar organic solvents, such as $C_1$-$C_4$ alkanols, dimethylsulfoxide (DMSO), dimethylformamide (DMF), and acetonitrile. The reaction is run from room temperature to reflux, preferably in an inert atmosphere, such as nitrogen.

Optically active ketones (Xa and Xb below) are used to prepare the optically active intermediates Vb and Vc

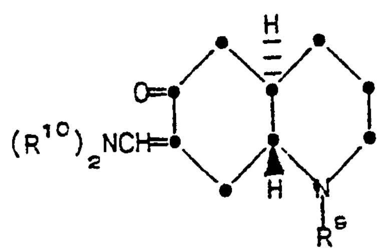

Vb

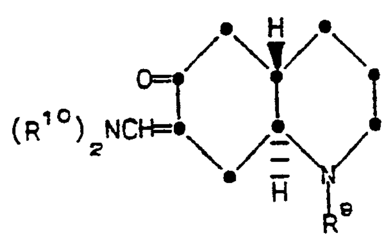

Vc

wherein $R^9$ and $R^{10}$ have the same significance as before. The preparation of the optically active ketone Xa

is described below. The preparation of the stereoisomeric ketone Xb is described below.

A similar synthetic route is used to prepare compounds according to formula I, II or IIa in which $R^2$ is other than H.

## Synthetic Route 2

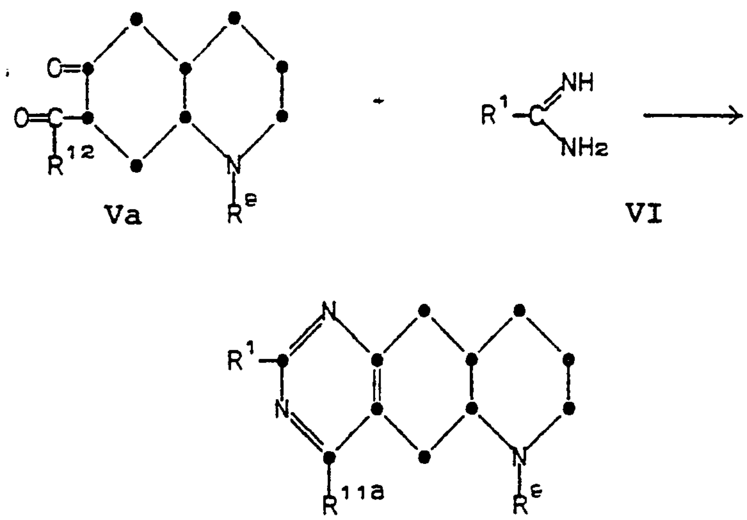

Va VI

Ia''

wherein $R^1$ and $R^9$ have their previous significance and $R^{12}$ is $CH_3$, or $C_1$-$C_3$ alkoxy, and $R^{11a}$ is $CH_3$ or OH. During the work-up of the ring closure reaction, the $R^{12}$ of the ester group is replaced with OH to yield a 4-OH derivative. This OH derivative is then halogenated to yield those compounds of formula I where $R^2$ is Cl or Br. Suitable halogenating agents are $POCl_3$, $PBr_3$, $SOCl_2$ or $SOBr_2$. The reaction is usually run at reflux temperature. An ether solvent can optionally be present.

The same procedure starting with the trans-(-)-enantiomer produces Id and with the trans-(+)-enantiomer produces Ie

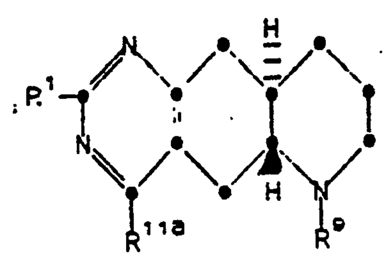

Id

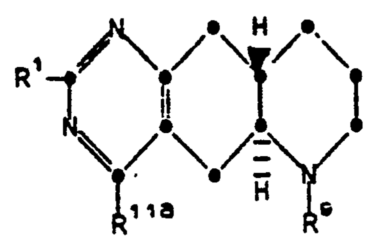

Ie

and this product is transformed where $R^{11a}$ is OH by halogenation to compounds of structure II or IIa.

The starting materials Va of synthetic route 2 are prepared according to the following procedure: the ketone (X for the trans racemate, Xa for the trans-(-)-stereoisomer and Xb for the trans-(+)-stereoisomer) is metallated at C-7 with a lithium amide, as for example lithium diisopropylamide ($LiN[CH(CH_3)_2]$), to form an enolate anion. This anion then reacts with acetyl chloride or an dialkylcarbonate to yield compounds represented by Va. These latter compounds are transformed to the desired pyrimidine, and then purified from contaminants.

Finally, compounds of formula I, II or IIa are most easily prepared by utilizing a ketone starting material (X, Xa or Xb)

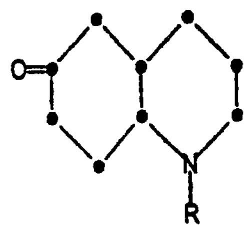

X

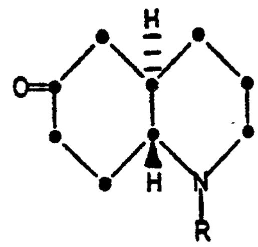

Xa

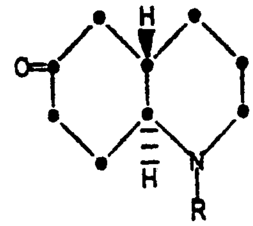

Xb

wherein R is $C_1$-$C_3$ alkyl or allyl. The ketones represented by X, when R is $C_1$-$C_3$ alkyl, are preferably prepared by the process whereby a 6-alkoxyquinoline of the formula

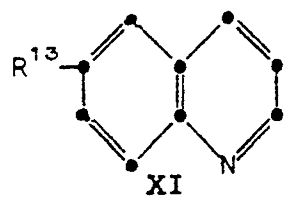

XI

wherein $R^{13}$ is $C_1$-$C_3$ alkoxy; is quaternized with a $C_1$-$C_3$ alkyl halide and the quaternized salt reduced to yield an N-$C_1$-$C_3$ alkyl-6-alkoxy-1,2,3,4-tetrahydroquinoline of the formula

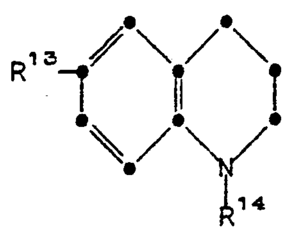

XIa

wherein $R^{14}$ is $C_1$-$C_3$ alkyl and $R^{13}$ is defined as above. The particular $C_1$-$C_3$ alkyl group remains intact through the next two reduction steps: a Birch reduction followed by a sodium cyanoborohydride or borohydride reduction to yield, ultimately, an octahydroquinoline of the formula XII

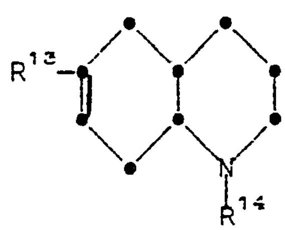

XII

wherein $R^{13}$ and $R^{14}$ are defined as above. This enol ether yields X (where R is $C_1$-$C_3$ alkyl) upon treatment with acid. Xa is then produced by resolution of X as previously set forth.

Compounds according to I, II or IIa in which R is allyl can also be prepared by a different procedure which is described below. The optically active ketones Xa and Xb are prepared by resolution of the trans-(±)-racemate as set forth below.

The ketones X or Xa are readily transformed by treatment with dimethylformamide dimethylacetal to yield V, the starting material of Synthetic Route 1.

An alternative preparation of the ketone X, when R is $C_1$-$C_3$ alkyl, is set forth in United States Patent 4,198,415.

An alternative intermediate to V, Vb or Vc, is a 1-alkyl-6-oxo-7-formyldecahydroquinoline disclosed in published European patent application 110496 as is the method of preparing this intermediate from the 6-oxo derivative (X, Xa or Xb).

An alternative intermediate to V, Vb or Vc, is a 1-alkyl-6-oxo-7-formyldecahydroquinoline disclosed below, as is the method of preparing this intermediate from the 6-oxo derivative (X, Xa or Xb).

The intermediates V, Vb and Vc can also be prepared by treating the above 1-alkyl-6-oxo-7-formyldecahydroquinoline with a primary or secondary amine in the presence of a dehydrating agent to yield the 7-mono- or dialkylaminomethylene derivative.

As can be seen from the above discussion, the group R in formula X carries through the synthetic procedure intact. Thus, if it is desired to replace one alkyl group with another or with allyl, indirect synthetic routes must be used. For example, if R in X, Xa or Xb is methyl or n-propyl, reaction with cyanogen bromideyields a 1-cyano-6-oxodecahydroquinoline XV, XVa or XVb where $R^{15}$ is cyano. Hydrolysis of the cyano group yields the secondary amine, XV, XVa or XVb where $R^{15}$ is H. Similarly, reaction of X, Xa or Xb where R is methyl with ethyl chloroformate yields an intermediate, XV, XVa or XVb, wherein $R^{15}$ is $C_2H_5$-O-CO, which can also be hydrolyzed to yield the corresponding compound XV, XVa or XVb wherein $R^{15}$ is H.

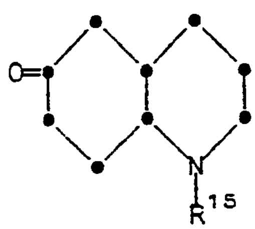

XV

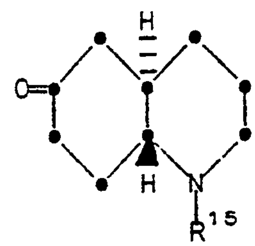

XVa

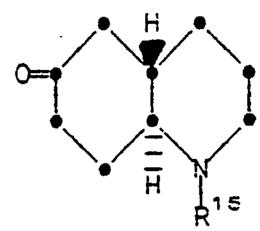

XVb

The secondary amine XV, XVa or XVb where $R^{15}$ is H can then be selectively alkylated, with the same or a different alkyl group, or can be allylated where it is desired to have an allyl group on the ring nitrogen. In this synthesis, the extremely reactive allyl halides can be used to ultimately yield X, Xa or Xb where R is allyl.

Again alternatively, XV, XVa or XVb in which $R^{15}$ is CN can be reacted with guanidine to yield a novel intermediate of formula III, IV or IVa, where $R^1$ is $NH_2$, $R^2$ is H, and $R^8$ is CN (IVb, IVc and IVd):

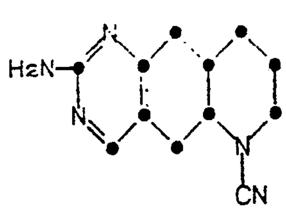

IVb

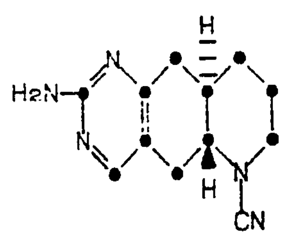

IVc

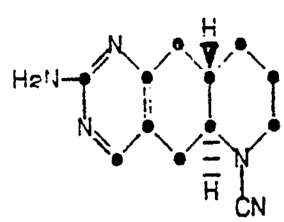

IVd

Hydrolysis of the cyano product yields compounds of formula III, IV or IVa in which $R^8$ and $R^2$ are both H and $R^1$ is $NH_2$. The hydrolysis is an acidic hydrolysis, such as aqueous hydrochloric acid, zinc chloride in acetic acid, or zinc in acetic acid, under standard conditions. Such compounds can then be selectively alkylated or allylated to yield drugs of formula I, II or IIa where $R^1$ is $NH_2$, $R^2$ is H, and R is as defined before. The alkylation (allylation) is done by conventional methods. For example an alkyl halide (or allyl halide) in a polar organic solvent, such as DMF; reduction amination in an alkanol or polar organic solvent, with an aldehyde and catalytic hydrogenation ($H_2$/Pd-on-carbon), formaldehyde/formic acid, or with an aldehyde and sodium cyanoborohydride with a trace of acid; lithium aluminum hydride or diborane

reduction of an amide formed by treating the above secondary amine with an acid halide or anhydride and a base, such as triethylamine or pyridine, and a solvent, such as ether, dioxane, tetrahydrofuran (THF), or dimethoxyethane (DME). This procedure is also a good method for introducing a tagged carbon into the R group at a late stage in the synthesis so as to avoid carrying the expensive (isotopic or radioactive) tagged molecule through several synthetic procedures with consequent loss of yield.

One still further synthetic route is available for preparing drugs in which R is allyl. This route involves adapting the Kornfeld-Bach synthesis disclosed in United States patent 4,198,415, Reaction Scheme I. By using an allyl halide in step 2 of the procedure of that patent, a trans-(±)-1-allyl-6-oxodecahydroquinoline is produced. This N-allyl derivative is then converted to trans-(±)-1-allyl-6-oxo-7-(dimethylaminomethylene)-decahydroquinoline, formula V wherein $R^9$ is allyl. The trans-(-)enantiomer or trans-(+)-enantiomer can then be produced from the racemate by resolution.

Compounds of formula I, II or IIa in which $R^1$ is $NHR^3$, $R^2$ is H, $CH_3$, Cl or Br, and $R^3$ is $R^6$-CO can be prepared by acylating Ia (or the 5aR,9aR stereoisomer or the 5aS,9aS stereoisomer) when $R^1$ is $NH_2$ with an acid chloride or anhydride under standard reaction conditions, such as a base (e.g. pyridine), a solvent (e.g. ether), and a catalyst (e.g. dimethylamine in pyridine).

Finally, there are two methods of providing the trans-(-) or 5aR,9aR derivatives, II or the trans-(+) or 5aS,9aS derivatives IIa. The first method is the resolution of the trans-(±) racemate (I) using a resolving agent such as d-(-)-tartaric acid or other suitable optically-active acid which forms a salt with the trans-(-) component of trans-(±)-2-substituted -6-$C_1$-$C_3$ alkyl (allyl)-5,5a,6,7,8,9,9a,10-octahydropyrimido[4,5-g]-quinoline. Similarly, use of a resolving agent of the opposite configuration, e.g. ℓ-(+)-tartaric acid, yields the 5aS,9aS-stereoisomer. Preferably, however, a resolution is carried out on the bicyclic ketone, X or XV, to produce 4aR,8aR-1-alkyl (or allyl)-6-oxodecahydroquinoline, using (-)-di-p-toluoyltartaric acid to form a salt with the 4aR,8aR-ketone or (+)-di-p-toluoyltartaric acid to form a salt with the 4aS,8aS ketone. The resolved ketone can then be reacted with dimethylformamide dimethylacetal, with tris(dimethylamino)methane or with ethyl formate followed by $(CH_3)_2NH$ to yield 4aR,8aR- (or 4aS,8aS)-1-alkyl (or allyl)-6-oxo-7-(disubstituted aminomethylene)decahydroquinoline of the formula Vb or Vc

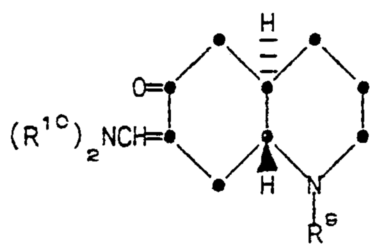

Vb

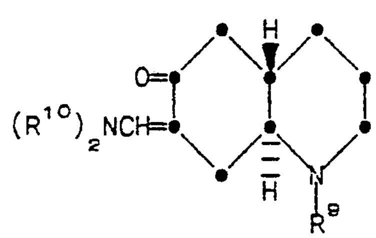

Vc

wherein $R^9$ and $R^{10}$ have their previously assigned meaning. Formula Vb or Vc can then be reacted with

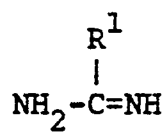

VI

wherein $R^1$ is defined as before, to yield the optically-active derivatives II or IIa directly or indirectly.

The above processes can yield salts. Conversion of the salt thus obtained to the corresponding free base is readily effected by dissolving the salt in water and then aiding an excess of an aqueous base (NaOH, $Na_2CO_3$ etc.). The free base, being insoluble in the basic solution, separates and is extracted with a water-immiscible organic solvent. The organic extract is then separated and dried. A solution containing one equivalent of a different non-toxic acid is then added, and the resulting salt isolated by filtration or by evaporation of the solvent. Alternatively, the solvent can be removed from the dried organic extract and the free base obtained as a residue. The free base can then be dissolved in a suitable solvent and the non-toxic acid added as a solution. The preferred salt is the HCl salt which can be prepared, for example, by adding an equivalent of ethanolic hydrogen chloride to an ethanolic solution of the free base, followed by evaporation of the ethanol and recrystallization of the residual salt. If it is desired to make a di salt such as a dihydrochloride salt, HCl gas can be passed into a solution of the free base to the point of saturation and

the di salt isolated as above.

Compounds represented by formula I, II or IIa each have two or more basic centers. The most basic of these is the octahydroquinoline ring amino group. This group forms salts readily with pharmaceutically-acceptable acids. Amine groups of lesser basicity are also present and these groups will form salts with strong pharmaceutically-acceptable inorganic acids, such as the mineral acids, or strong organic acids such as p-toluenesulfonic acid, to yield di salts. Pharmaceutically-acceptable acid addition salts thus include mono or di salts derived from inorganic acids such as those listed earlier in this application.

This invention is further illustrated by the following specific examples.

STARTING MATERIALS AND INTERMEDIATES

Example A

Preparation of 4aR,8aR-1-n-propyl-6-oxodecahydroquinoline

Ten g. of (-)-di-p-toluoyltartaric acid were dissolved in 75 ml. of warm methanol. The solution was added to a solution of 5.05 g. of trans-dℓ-1-n-propyl-6-oxodecahydroquinoline in 15 ml. of methanol. The reaction mixture was brought to a boil and was then allowed to cool to ambient temperature. After remaining at ambient temperature overnight, crystallization was induced by the addition of seed crystals previously obtained. The crystalline tartarate salt was isolated by filtration and the filter cake washed with methanol; yield = 2.813 g. (18.7%) of a white crystalline solid comprising the (-)-di-p-toluoyltartrate of 4aR,8aR-1-n-propyl-6-oxodecahydroquinoline; $[\alpha]_D^{25°}$ = -107.49° (MeOH, c = 1). Recrystallization of the salt from methanol gave 1.943 g. of the optically pure salt, $[\alpha]_D^{25°}$ = -108.29° (MeOH, c = 1).

The (-)-di-p-toluoyltartrate salt thus obtained was treated with dilute aqueous sodium hydroxide and the resulting alkaline solution extracted with methylene dichloride. The methylene dichloride extract was dried, concentrated and the solvent removed therefrom in vacuo. The resulting residue was distilled to yield a colorless oil comprising purified 4aR,8aR-1-n-propyl-6-oxodecahydroquinoline; $[\alpha]_D^{25°}$ = -88.51° (MeOH, c = 1).

Other 1-(alkyl, allyl, benzyl, or cyano)-6-oxodecahydroquinolines can be resolved in a similar manner.

Example B

Preparation of 4aS,8aS-1-n-propyl-6-oxodecahydroquinoline

A resolution of trans-(±)-1-n-propyl-6-oxodecahydroquinoline was carried out according to the following procedure: Ten grams of (-)-di-p-toluoyltartaric acid were dissolved in 75 ml. of warm methanol. The solution was added to a solution of 5.05 g. of trans-[±]-1-n-propyl-6-oxodecahydroquinoline in 15 ml. of methanol. The reaction mixture was brought to a boil and was then allowed to cool to ambient temperature. After remaining at ambient temperature overnight, crystallization was induced by the addition of seed crystals previously obtained. The crystalline tartarate was isolated by filtration and the filter cake washed with methanol; yield = 2.813 g. (18.7%) of a white crystalline solid comprising the (-)-di-p-toluoyltartrate of 4aR,8aR-1-n-propyl-6-oxodecahydroquinoline; $[\alpha]_D^{25°}$ = -107.49° (MeOH, c = 1).

Filtrates and mother liquors from the above procedure or from similar, larger scale procedures containing tartrates were combined and the combined solutions treated with alkali, thus forming the free bases which were extracted into a water-immiscible solvent to yield a solution of 1-n-propyl-6-oxodecahydroquinoline enriched as regards the 4aS,8aS-isomer, and depleted as regards the 4aR,8aR isomer. The solution was treated with (+)-ditoluoyl tartaric acid monohydrate, in accordance with the above procedure, to yield 4aS,8aS-1-n-propyl-6-oxodecahydroisoquinoline-(+) ditoluoyl tartrate of about 80% ee optical purity (ee = enantiomeric excess). 20 g. of the salt were crystallized from 250 ml. of methanol to give 12 g. of a white crystalline powder melting at 167.5-169.5° C. with decomposition;

$$[\alpha]_{D}^{25} = +106.3°$$

(methanol, c = 1.0);

$$[\alpha]_{365}^{25} = +506.7°$$

(methanol, c = 1.0). These figures indicate an optical purity of about 90% ee. A second crop obtained from mother liquors from the above crystallization gave 2.3 g. of a white solid melting at about 166.0-166.5° C. with decomposition; $[\alpha]_{D}^{25}$ = 106.6°;

$$[\alpha]_{365}^{25} = +510.8°$$

(methanol, c = 1.0 for both) indicating optical purity of about 94% ee. Recrystallization of first and second crop crystals from methanol gave a white solid from which the free base was obtained by standard procedures. The free base was distilled to yield 4.14 g. of a colorless oil boiling at 82-86° C. at 0.13 torr., comprising 4aS,8aS-1-n-propyl-6-oxodecahydroquinoline; $[\alpha]_{D}^{25}$ = +86.2°;

$$[\alpha]_{365}^{25} = +376.6°$$

(methanol, c = 1.0 for both rotations); optical purity = about 98% ee.

Alternatively, trans-(±)-1-n-propyl-6-oxodecahydroquinoline can be treated directly with (+)-di-p-toluoyltartaric acid to yield the 4aS,8aS-1-n-propyl-6-oxodecahydroquinoline-(+)-di-p-toluoyltartrate which is purified by the procedures set forth above.

Example C

Preparation of trans-(±)-1-n-propyl-6-oxo-7-ethoxycarbonyldecahydroquinoline

A suspension of 790 mg. of sodium hydride (55% in mineral oil) was placed in a 50 ml. round bottom flask and the mineral oil removed by three hexane washes. The solid residual sodium hydride was suspended in 8 ml. of THF and 1.45 ml. (1.41 g.) of diethyl carbonate added along with one drop of anhydrous ethanol. The resulting solution was heated to refluxing temperature and 1.1 g. of trans-(±)-1-n-propyl-6-oxodecahydroquinoline in 5 ml. of THF was added over a five minute period. The resulting mixture was heated to reflux temperature overnight. TLC at this point indicated no remaining starting material and a new, slower moving spot was present. The reaction mixture was poured into water giving a pH of the aqueous layer of about 14. The alkaline layer was extracted with methylene dichloride. The pH of the aqueous layer was adjusted to about pH = 9 and again the alkaline layer was extracted with methylene dichloride. The methylene dichloride extracts were combined and the combined extracts dried and the solvent removed therefrom to yield 1.56 g. of a yellow oil comprising trans-(±)-1-n-propyl-6-oxo-7-ethoxycarbonyldecahydroquinoline formed in the above reaction. Chromatography of the residue over Woelm silica (100-200 mesh) using a 1:1 ether/hexane solvent mixture containing a trace of 14N aqueous ammonium hydroxide as the eluant. Fractions containing the desired product were combined to yield eventually 880 mg. (55% yield) of a yellow oil. The keto ester was shown by nmr to exist in an enol form represented by the following structure.

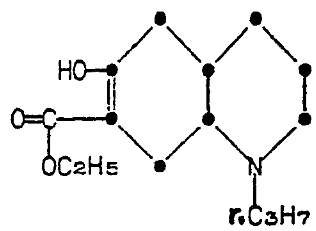

The compound had the following nmr spectrum: nmr ($CDCl_3$): 12.20 (s, 1H); 4.28 (q, J=7, 2H); 3.20-1.10 (m, 16H); 1.36 (t, J=7, 3H); 0.95 (t, J=7, 3H)

Example D

Preparation of trans-(±)-1-n-propyl-6-oxo-7-dimethylaminomethylenedecahydroquinoline

Four grams of trans-(±)-1-n-propyl-6-oxodecahydroquinoline were added to a solution of 5.6 g. of potassium t-butoxide in about 50 ml. of anhydrous redistilled tetrahydrofuran. The reaction mixture was stirred for 30 minutes under a nitrogen atmosphere. Next, 3.6 ml. of ethyl formate were added in dropwise fashion while the reaction mixture was cooled in an ice-alcohol bath. After the addition had been completed, the reaction mixture was stirred at ambient temperature under a nitrogen atmosphere overnight. The reaction mixture, at this point a solid slurry, was neutralized with glacial acetic acid. Methanol was added to the slurry, followed by 1 ml. of dimethylamine. 3A molecular sieves was added to aid dehydration. The subsequent reaction mixture was stirred under a nitrogen atmosphere for 48 hours, and was then filtered. The filtrate was evaporated to dryness in vacuo. Water was added and the aqueous mixture extracted three times with equal volumes of methylene dichloride. The methylene dichloride extracts were combined and the combined extracts washed with water and then dried. Evaporation of the methylene dichloride yielded 4.15 g. (81.4%) yield of trans-(±)-1-n-propyl-6-oxo-7-dimethylaminomethylenedecahydroquinoline.

Example E

Preparation of trans-(±)-2-amino-6-cyano-5,5a,6,7,8,9,9a,10-octahydropyrimido[4,5-g]quinoline

A reaction mixture was prepared from 16 g. of trans-(±)-1-methyl-6-oxodecahydroquinoline (prepared by the procedure of Bach and Kornfeld U.S. Patent 4,198,415), 26 g. of cyanogen bromide and 450 ml. of methylene dichloride. The reaction mixture was stirred overnight at room temperature and was then extracted three times with 1N aqueous hydrochloric acid. The acid extracted reaction mixture was washed with saturated aqueous sodium bicarbonate and then dried. Any volatile material were removed by evaporation in vacuo. The residue thus obtained was 18.8 g. of a semi-solid oil comprising trans-(±)-1-cyano-6-oxodecahydroquinoline formed in the above reaction. Chromatography of the oil over Florisil using chloroform as the eluant yielded fractions of purified material weighing collectively 11.5 g. (66%) yield. The oil crystallized upon standing to yield white crystals.

A reaction mixture was prepared from 4.18 g. of trans-(±)-1-cyano-6-oxodecahydroquinoline, 5.0 g. of tris-dimethylaminomethane and 50 ml. of toluene. The reaction mixture was refluxed under nitrogen temperature for five hours and then was concentrated in vacuo. Five and seventy-six hundredths grams of a crude yellow solid comprising trans-(±)-1-cyano-6-oxo-7-dimethylaminomethylenedecahydroquinoline were obtained. This crude product was mixed with 2.25 g. of guanidine carbonate in 100 ml. of anhydrous methanol. This reaction mixture was heated to reflux under nitrogen overnight, and was then concentrated in vacuo. The resulting solid residue was triturated with hot methanol and filtered. The filter cake was washed twice with methanol and once with ether. A yield of 4.11 g. (78%) of trans-(±)-2-amino-6-cyano-5,5a,6,7,8,9,9a,10-octahydropyrimido[4,5-g]quinoline were obtained having the following physical characteristics. Mass spectrum, molecular ion at 229; infrared spectrum peaks ($cm^{-1}$) at 3307.18, 3157.70, 2202.87, 1660.83, 1599.10, 1564.38, 1486.26.

Analysis Calculated: C, 62.86; H, 6.59; N, 30.54;
Found: C, 63.18; H, 6.70; N, 30.24.

Example F

Preparation of trans-(±)-2-amino-5,5a,6,7,8,9,9a,10-octahydropyrimido[4,5-g]quinoline

A reaction mixture was prepared from 1.66 g. of the 6-cyano compound of Example E, 9.7 g. of zinc dust, 200 ml. of acetic acid and 50 ml. of water. The reaction mixture was heated to reflux temperature under a nitrogen atmosphere for about 24 hours and then stirred at room temperature for a 48 hour period. Any volatile material was removed from the reaction mixture in vacuo and the resulting residue dissolved in water. The aqueous mixture was made basic with 50% aqueous sodium hydroxide. (eventual pH was in the range 10-11). A heavy white precipitate formed. The basic solution was filtered and the filtrate extracted three times with a 3:1 by volume chloroform/isopropanol solvent mixture. The organic extracts were combined and dried. Removal of the solvent in vacuo yielded 0.43 g. of a light yellow powder comprising trans-(±)-2-amino-5,5a,6,7,8,9,9a,10-octahydropyrimido[4,5-g]quinoline free base. The free base was converted to the hydrochloride salt which was recrystallized from a methanol/acetate solvent mixture to yield crystalline material, MP = above 230° C.

Analysis (after drying at 150°C.)
Calculated: C, 47.66; H, 6.55; N, 20.21;
Found: C, 47.37; H, 6.65; N, 19.91.

Trans-(±)-2-amino-5,5a,6,7,8,9,9a,10-octahydropyrimido[4,5-g]quinoline thus prepared can be alkylated with a lower alkyl halide or allylated with an allyl halide to yield compounds coming within the scope of formula I above.

Some of the above preparations were carried out with the racemate. It will be apparent to those skilled in the art that these same chemical steps can be carried out on the separated trans-(-) or trans-(+)-stereoisomers to yield optically active intermediates and final products.

Example G

Preparation of trans-(±)-2-Amino-4-hydroxy-6-n-propyl-5,5a,6,7,8,9,9a,10-octahydropyrimido[4,5-g]quinoline

A reaction mixture was prepared from 2.0 g. of trans-(±)-1-n-propyl-6-oxo-7-ethoxycarbonyldecahydro quinoline (prepared in Example C), 20 ml. of anhydrous ethanol and 0.67 g. of guanidine carbonate. The reaction mixture was heated to reflux temperature overnight under a nitrogen atmosphere. The white precipitate which formed was collected by filtration and the filter cake washed with ethanol and dried; yield = 1.36 g. The filter cake was dissolved in 52 ml. of 0.1N aqueous hydrochloric acid. The acidic mixture was filtered and the filtrate concentrated in vacuo. The solid residue was dissolved in boiling methanol. The methanol solution was filtered and trans-(±)-2-amino-4-hydroxy-6-n-propyl-5,5a,6,7,8,9,9a,10-octahydropyrimido[4,5-g]quinolinehydrochloride thus prepared crystallized to yield 0.79 g. of product. The free base had the following physical characteristics: mass spectrum, molecular ion at 262.

Analysis Calculated: C, 64.09; H, 8.45; N, 21.36;
Found: C, 64.18; H, 8.51; N, 21.13.

The hydrochloride salt had the following physical characteristics: mass spectrum, molecular ion at 262.

FINAL PRODUCTS

Example 1

Preparation of trans-(±)-2-amino-6-methyl-5,5a,6,7,8,9,9a,10-octahydropyrimido[4,5-g]quinoline

A reaction mixture was prepared from 1.8 g. of trans-(±)-1-methyl-6-oxodecahydroquinoline and 2.2 g. of tris-dimethylaminomethane in 18 ml. of toluene. The reaction mixture was refluxed under nitrogen for about 12 hours. An additional 0.8 g. of tris-dimethylaminomethane were added and refluxing continued under nitrogen for an additional 5 hours. The reaction mixture was then concentrated to dryness in vacuo. The resulting residue containing trans-(±)-1-methyl-6-oxo-7-(dimethylaminomethylene)decahydroquinoline formed in the above reaction was dissolved in 40 ml. of ethanol to which was added 1.5 g. of guanidine carbonate. The resulting mixture was heated overnight to reflux temperature under a nitrogen atmosphere. On cooling, a crystalline precipitate formed which was collected by filtration and the filter cake washed with ethanol; yield = 0.68 g. (38%) of a light yellow powder. The material was dissolved in 1N aqueous hydrochloric acid. The acidic solution was then made basic with 10% aqueous sodium hydroxide. Trans-(±)-2-amino-6-methyl-5,5a,6,7,8,9,9a,10-octahydropyrimido[4,5-g]quinoline free base, being insoluble in the alkaline layer, separated and was extracted with chloroform. The chloroform extract was dried and the chloroform removed in vacuo. The residue, comprising trans-(±)-2-amino-6-methyl-5,5a,6,7,8,9,9a,10-octahydropyrimido[4,5-g]quinoline, was suspended in ethanol and the ethanol solution saturated with gaseous hydrogen chloride. The solvent was removed in vauco and the resulting residue, the dihydrochloride salt of trans-(±)-2-amino-6-methyl-5,5a,6,7,8,9,9a,10-octahydropyrimido[4,5-g]quinoline, was recrystallized from hot ethanol. Sixty-six mg. of dihydrochloride salt were obtained, MP = 262-275 °C. (decomposition) and having the following analysis (after drying at 150 °C.):

| | |
|---|---|
| Theory: | C, 49.49; H, 6.92; N, 19.24 |
| Found: | C, 49.61; H, 7.03; N, 18.92 |

The higher temperature drying was necessary because it became apparent after drying at lower temperatures that the dihydrochloride salt crystallizes as a solvate and the solvent must be removed by drying to obtain a reproducible analysis.

Example 2

Preparation of trans-(±)-2-amino-6-n-propyl-5,5a,6,7,8,9,9a,10-octahydropyrimido[4,5-g]quinoline

The reaction of Example 1 was repeated except that 1 g. of trans-(±)-1-n-propyl-6-oxo-7-dimethylaminomethylenedecahydroquinoline was reacted with 0.4 g. of guanidine carbonate in 20 ml. of anhydrous ethanol. (Trans-(±)-1-n-propyl-6-oxo-7-dimethylaminomethylenedecahydroquinoline was prepared from trans-(±)-1-n-propyl-6-oxodecahydroquinoline and tris-dimethylaminomethane according to the above procedure). The reaction mixture was heated under reflux temperature overnight at which time a precipitate was observed. The reaction mixture was chilled in an ice bath and a light yellow crystalline precipitate comprising trans-(±)-2-amino-6-n-propyl-5,5a,6,7,8,9,9a,10-octahydropyrimido[4,5-g]quinoline formed in the above reaction was collected. The filter cake was washed with ethanol and then dried; MP = above 260 °C. Yield = 0.6 g. (61%).

| | |
|---|---|
| Analysis calculated: | C, 68.26; H, 9.00; N, 22.74 |
| Found: | C, 68.45; H, 8.87; N, 22.26 |

Trans-(±)-2-amino-6-n-propyl-5,5a,6,7,8,9,9a,10-octahydropyrimido[4,5-g]quinoline was dissolved in 1N aqueous hydrochloric acid and the acidic solution extracted with ether. The acidic solution was then made basic with 10% aqueous sodium hydroxide. Trans-(±)-2-amino-6-n-propyl-5,5a,6,7,8,9,9a,10-octahydropyrimido[4,5-g]quinoline precipitated and was separated by filtration. The free base was dissolved in 1N aqueous hydrochloric acid, and the water removed in vacuo. The resulting residue was recrystallized from hot ethanol. Yield = 0.54 g. (40%). Trans-(±)-2-amino-6-n-propyl-5,5a,6,7,8,9,9a,10-octahydropyrimido-[4,5-g]quinoline dihydrochloride thus prepared had a MP = 225-270°C. and had the following analysis.

Analysis calculated for $C_{14}H_{22}N_4 \cdot 2HCl \cdot H_2O$

C, 49.74; H, 7.75; N, 16.57; Cl, 20.97;

Found: C, 49.88; H, 8.03; N, 16.81; Cl, 20.87.

After drying at 120°C., analysis indicated that water of hydration and one-half mole of hydrogen chloride had been lost to yield trans-(±)-2-amino-6-n-propyl-5,5a,6,7,8,9,9a,10-octahydropyrimido[4,5-g]-quinoline sesquihydrochloride having the following analysis.

Analysis calculated for $C_{14}H_{22}N_4 \cdot 1.5\ HCl$

C, 55.86; H, 7.87; N, 18.61; Cl, 17.03

Found: C, 55.49; H, 7.83; N, 18.35; Cl, 17.03.

Example 3

Preparation of 5aR,9aR-2-Amino-6-n-propyl-5,5a,6,7,8,9,9a,10-octahydropyrimido[4,5-g]quinoline

Following the procedure of Example 1, 4aR,8aR-1-n-propyl-6-oxo-7-dimethylaminomethylenedecahydroquinoline (prepared from 4aR,8aR-1-n-propyl-6-oxodecahydroquinoline and tris-dimethylaminomethane shown in Example A) was reacted with guanidine carbonate in anhydrous ethanol solution. The reaction was carried out and the reaction mixture worked up as in Example 1 to yield 2.4 g. of 5aR,9aR-2-amino-6-n-propyl-5,5a,6,7,8,9,9a,10-octahydropyrimido[4,5-g]quinoline.

The product was suspended in ethanol and gaseous hydrogen chloride bubbled through the suspension. The resulting solution was evaporated to dryness in vacuo and the residual yellow oil dissolved in about 10 ml. of ethanol. Ether was added to the point of incipient precipitation and the mixture heated on the steam bath. Upon cooling, fine, powdery crystals formed which were separated by filtration. The filter cake was washed with ethanol to yield .72 g. of the dihydrochloride salt of 5aR,9aR-2-amino-6-n-propyl-5,5a,6,7,8,9,9a,10-octahydropyrimido[4,5-g]quinoline.

Analysis (after drying at 180°C.)

C, 52.67; H, 7.58; N, 17.55

Found: C, 52.81; H, 7.75; N, 17.65.

Molecular ion at 246;

Optical rotation $[\alpha]_{589}^{25°C.} = -99.6°$;

$[\alpha]_{360}^{25°C.} = -374.8°$

Example 4

Preparation Trans-(±)-2-dimethylamino-6-n-propyl-5,5a,6,7,8,9,9a,10-octahydropyrimido[4,5-g]quinoline

A reaction mixture was prepared from 4.7 g. of trans-(±)-1-n-propyl-6-oxo-7-dimethylaminomethylenedecahydroquinolineand 2.5 g. of N,N-dimethylguanidine hydrochloride in 50 ml. of anhydrous ethanol. The reaction mixture was heated overnight under a nitrogen atmosphere, and was then cooled and the volatile constituents removed in vacuo. The resulting residue was dissolved in ethyl acetate and the ethyl acetate solution contacted with an excess of 10% aqueous sodium hydroxide. Trans-(±)-2-dimethylamino-6-n-propyl-5,5a,6,7,8,9,9a,10-octahydropyrimido[4,5-g]quinoline formed in the above reaction, being insoluble in the basic layer, remained in the ethyl acetate layer. The aqueous layer was separated and the ethyl acetate layer extracted once with water and once with saturated aqueous sodium chloride. The ethyl acetate layer was dried and the ethyl acetate removed in vacuo to leave 0.75 g. of an orange oil. The oily residue was chromatographed over Florisil using hexane containing increasing amounts (1-50% by volume) of ethyl acetate as the eluant. Fractions shown by TLC to contain the desired trans-(±)-2-dimethylamino-6-n-propyl-5,5a,6,7,8,9,9a,10-octahydropyrimido[4,5-g]quinoline were combined and the solvent removed from the combined fractions in vacuo. The resulting residue was dissolved in ethanol and gaseous hydrogen chloride passed into the solution thus forming the corresponding dihydrochloride salt. The ethanol was removed therefrom in vacuo and the dihydrochloride salt crystallized from a methanolethyl acetate solvent mixture to yield 0.170 g. of a white solid having a molecular ion at 274 and MP = above 250° C.

| | |
|---|---|
| Analysis calculated: | C, 55.33; H, 8.13; N, 16.13 |
| Found: | C, 55.67; H, 8.19; N, 16.19. |

Example 5

Preparation of trans-(±)-2-methylamino-6-n-propyl-5,5a,6,7,8,9,9a,10-octahydropyrimido[4,5-g]quinoline

Following the procedure of Example 4, but substituting N-methylguanidine for N,N-dimethylguanidine, trans-(±)-2-methylamino-6-n-propyl-5,5a,6,7,8,9,9a,10-octahydropyrimido[4,5-g]quinoline was prepared. The compound was purified by chromatography over Florisil using methylene dichloride containing increasing (0-10% by volume) methanol as the eluant; yield = 0.66 g. The monohydrochloride salt was prepared by adding an equivalent of 0.1N hydrochloric acid to the solid and recrystallizing the product from methanol; yield = 599 mg. MP = above 240° C.

| | |
|---|---|
| Analysis calculated: | C, 60.69; H, 8.49; N, 18.87; Cl, 11.94 |
| Found: | C, 60.96; H, 8.53; N, 19.07; Cl, 11.74. |

In Examples 1,2,4 and 5, the optically active 5aR,9aR or 5aS,9aS derivatives can be prepared from the desired 4aR,8aR- (or 4aS,8aS)-$C_1$-$C_3$ alkyl-6-oxo-7-dimethylaminomethylene decahydroquinoline and a suitable guanidine.

Example 6

Preparation of trans-(±)-2-Amino-4-methyl-6-n-propyl-5,5a,6,7,8,9,9a,10-octahydropyrimido[4,5-g]quinoline

A reaction mixture was prepared by adding 13.7 ml. of 1.6M n-butyllithium in hexane to a solution containing 3.1 ml. of diisopropylamine and 22 ml. of THF at about 0° c. under a nitrogen atmosphere. The reaction mixture was stirred for about 30 minutes. Next, 2.0 g. of trans-(±)-1-n-propyl-6-oxodecahydroquinoline in a small amount of THF was added while containing the reaction mixture at about -78° C. The solution was stirred for two hours at which time 1.1 ml. of acetyl chloride was added. This new reaction mixture was stirred at about -78° C. for about 30 minutes and then at room temperature for two hours. The reaction mixture was next poured into water and the consequent aqueous mixture acidified to a pH = 9-10 with 1N aqueous hydrochloride acid. The aqueous solution was extracted three times with equal volumes of methylene dichloride. The methylene dichloride extracts were combined and the combined extracts dried. Evaporation of the solvent yielded 2.7 g. of trans-(±)-1-n-propyl-6-oxo-7-acetyldecahydroquinoline. The crude reaction product (without further purification) was mixed with about 0.9 g. of guanidine carbonate. Forty ml. of ethanol were added and the reaction mixture refluxed under a nitrogen atmosphere. The reaction mixture was then evaporated to dryness and the crude product chromatographed over Florisil. Fractions shown to contain trans-(±)-2-amino-4-methyl-6-n-propyl-5,5a,6,7,8,9,9a,10-octahydropyrimido[4,5-g]quinoline formed in the above reaction were combined to yield 270 mg of free base, and 10 ml. of 0.1N aqueous hydrochloric acid were added thereto. The dihydrochloride salt thus formed was recrystallized from ethanol; m.p. = above 240° C; mass spectrum molecular ion at 260, small peak at 268.

| | |
|---|---|
| Analysis Calculated: | C, 54.05; H, 7.86; N, 16.81; |
| Found: | C, 53.93; H, 7.98; N, 16.61. |

Example 7

Preparation of trans-(±)-2-amino-4-chloro-6-n-propyl-5,5a,6,7,8,9,9a,10-octahydropyrimido[4,5-g]quinoline

The 4-hydroxy product obtained in Example G was refluxed with 4 ml. of phosphorous oxychloride. The reaction mixture, containing trans-(±)-2-amino-4-chloro-6-n-propyl-5,5a,6,7,8,9,9a,10-octahydropyrimido[4,5-g]quinoline formed in the above reaction, was poured onto ice and the resulting aqueous mixture made basic. The basic mixture was filtered and the insoluble material (30 mg.) dissolved in 0.1N aqueous hydrochloric acid. The hydrochloride salt thus prepared was recrystallized from ethanol to yield 13.6 mg. of trans-(±)-2-amino-4-chloro-6-n-propyl-5,5a,6,7,8,9,9a,10-octahydropyrimido[4,5-g]quinolinehydrochloride having the following physical characteristics. Mass spectrum, molecular ion at 280, smaller peak at 282.

| | |
|---|---|
| Analysis Calculated: | C, 53.00; H, 6.99; N, 17.66; |
| Found: | C, 53.15; H, 6.92; N, 17.77. |

The 4-bromo derivative can be made similarly by substituting $PBr_3$ for $POCl_3$ in the above reaction.

Example 8

Preparation of Trans-(±)-2-acetylamino-6-n-propyl-5,5a,6,7,8,9,9a,10-octahydropyrimido[4,5-g]quinoline

A solution was prepared containing 0.75 g of trans-(±)-2-amino-6-n-propyl-5,5a,6,7,8,9,9a,10-octahydropyrimido[4,5-g]quinoline in 20 ml. of pyridine and 0.34 g. of acetic anhydride was added thereto in dropwise fashion. The reaction mixture was heated to reflux temperature under a nitrogen blanket overnight. TLC at this point in time indicated that starting material was still present; therefore, about 1.5 ml. more

acetic anhydride were added and the reaction mixture again heated to reflux temperature under a nitrogen blanket. TLC, using a 9:1 by volume chloroform/methanol solvent system containing ammonia, indicated that the reaction had gone largely toward completion but that some startingmaterial was still present. The reaction mixture was therefore concentrated in vacuo and the resulting residue triturated in hot ethyl acetate. On cooling, crystals formed which were isolated by filtration, yielding 340 mg. of trans-(±)-2-acetylamino-6-n-propyl-5,5a,6,7,8,9,9a,10-octahydropyrimido[4,5-g]quinoline. $R_f$ at 0.7; Molecular ion at 288; nmr and infrared spectra were in comformance with the proposed structure.

Example 9

Preparation of trans-(±)-2-benzoylamino-6-n-propyl-5,5a,6,7,8,9,9a,10-octahydropyrimido[4,5-g]quinoline

Following the procedure of Example 8, trans-(±)-2-amino-6-n-propyl-5,5a,6,7,8,9,9a,10-octahydropyrimido[4,5-g]quinoline was reacted with benzoyl chloride in pyridine solution. The residue of 450 mg. of yellow-orange oil obtained after working up the reaction mixture as indicated above was chromatographed over Florisil using chloroform with increasing amounts (0-10% by volume) of methanol as the eluant. Fraction ten contained the desired 2-benzoylamino compound (shown by TLC). The solvent was removed therefrom in vacuo. The resulting residue was dissolved in ethanol and gaseous hydrogen chloride passed into the ethanol solution. Addition of ether to the point of incipient precipitation yielded trans-(±)-2-benzoylamino-6-n-propyl-5,5a,6,7,8,9,9a,10-octahydropyrimido[4,5-g]quinolinedihydrochloride; molecular ion at 350.

Analysis (after drying at 130°C.)

C, 59.57; H, 6.67; N, 13.23

Found: C, 59.35; H, 6.85; N, 12.99.

Example 10

Preparation of 5aS,9aS-2-amino-6-n-propyl-5,5a,6,7,8,9,9a,10-octahydropyrimido[4,5-g]quinoline

A solution was prepared from 3.37 g. of 4aS,8aS-1-n-propyl-6-oxodecahydroquinoline (prepared in Example B) in 60 ml. of toluene. Six and twenty-six hundredths grams (7.5 ml.) of tris(dimethylamino)-methane were added in dropwise fashion. The resulting mixture was heated to reflux temperature for about four hours, at which time tlc indicated no spot corresponding to starting material. Concentration of the reaction mixture yielded 4.813 g. of a yellow oil which was chromatographed over a 50 mm. X 30 cm. silica gel column using 8% methanol in methylene dichloride plus concentrated ammonium hydroxide as the eluant. Fractions shown by tlc to contain 4aS,8aS-1-n-propyl-6-oxo-7-dimethylaminomethylenedecahydroquinoline were combined to give 3.651 g. of a yellow oil. This material, without further purification, was dissolved in 30 ml. of ethanol, and the solution was added to a suspension of 2.56 g. of guanidine carbonate in 70 ml. of anhydrous ethanol. The reaction mixture was heated to refluxing temperature for about 18 hours, after which time it was cooled in an ice bath. The precipitate which had formed was collected by filtration to yield 3.506 g. of fine, light yellow needles comprising a salt of 5aS,9aS-2-amino-6-n-propyl-5,5a,6,7,8,9,9a,10-octahydropyrimido[4,5-g]quinoline. The salt thus obtained was converted to the dihydrochloride salt by standard procedures. The dihydrochloride salt was dissolved in water. The resulting acidic aqueous solution was made basic with aqueous sodium hydroxide. The free base, being insoluble in the alkaline layer, separated and was extracted into methylene dichloride. Evaporation of the extract to dryness yielded a white foam which was dissolved in a 1:1 methanol/methylene dichloride solvent mixture and the solution saturated with gaseous HCl. Concentration of the solution yielded a yellow foam which was recrystallized from methanol/ethyl acetate to yield a white powder comprising the dihydrochloride salt of 5aS,9aS-2-amino-6-n-propyl-5,5a,6,7,8,9,9a,10-octahydropyrimido[4-5-g]quinoline having the following elemental analysis:

Calculated: C, 52.67; H, 7.58; N, 17.55; Cl, 22.21;
Found: C, 52.39, H, 7.36, N, 17.31, Cl, 22.40,

$[\alpha]_D^{25}$ = +108.3°, $[\alpha]_{25}^{365}$ = +405.2° (both in methanol, c = 1.0).

As previously stated the compounds of formulae I and II above are dopamine D-2 agonists without any other pronounced pharmacologic action. One of such dopamine D-2 agonist activities is the inhibition of prolactin secretion, as demonstrated according to the following procedure.

Adult male rats of the Sprague-Dawley strain weighing about 200 g. were housed in an air-conditioned room with controlled lighting (lights on 6 a.m. - 8 p.m.) and fed lab chow and water ad libitum. Each rat received an intraperitoneal injection of 2.0 mg. of reserpine in aqueous suspension 18 hours before administration of the test drug. The purpose of the reserpine was to keep the rat prolactin levels uniformly elevated. The test compound was dissolved in 10 percent ethanol, and injected intraperitoneally at doses from 100 μg./kg. to 1 μg./kg. The test compound was administered at each dose level to a group of 10 rats, and a control group of 10 intact males received an equivalent amount of 10 percent ethanol. One hour after treatment, all rats were killed by decapitation, and 150 μl aliquots of serum were assayed for prolactin.

The difference between the prolactin level of the treated rats and prolactin level of the control rats, divided by the prolactin level of the control rats gives the percent inhibition of prolactin secretion attributable to the given dose. Inhibition percentages are given in Tables 1 and 2 below for compounds of formulae I or II above, respectively. In the tables, columns 1, 2 and 3 give substitution patterns for the basic structures at the head of the Table, column 4 the form (salt or free base--FB), column 5 the route of administration, and columns 6, 7, 8, and 9 the percent prolactin inhibition at the specified dose levels. In some instances a compound was tested more than once at a particular dose level and the present prolactin inhibition figures in the tables are averages from these multiple tests.

Table 1

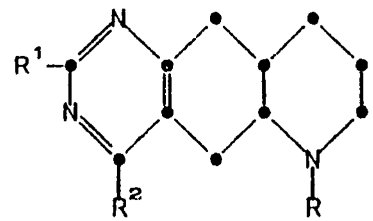

I

| R | $R^1$ | $R^2$ | Form | Route | 100 µg/kg | 50 µg/kg | 10 µg/kg | 5 µg/kg | 1 µg/kg |
|---|---|---|---|---|---|---|---|---|---|
| $CH_3$ | $NH_2$ | H | 2 HCl | IP | - | 22 | 23 | -- | -- |
| $n-C_3H_7$ | $NH_2$ | H | 2 HCl | IP | 94 | 85 | 72 | -- | 13 |
| $n-C_3H_7$ | $NH_2$ | H | 2 HCl | oral | -- | 91 | 74 | 71 | 44 |
| $n-C_3H_7$ | $C_6H_5CONH$ | H | FB | IP | -- | 78 | 32 | -- | -- |
| $n-C_3H_7$ | $CH_3CONH$ | H | FB | IP | -- | 92 | 63 | -- | -- |
| $n-C_3H_7$ | $(CH_3)_2N$ | H | 2 HCl | IP | -- | 81 | -- | 21 | -- |
| $n-C_3H_7$ | $NH_2$ | Cl | HCl | IP | -- | 36 | -- | -- | -- |
| $n-C_3H_7$ | $NH_2$ | $CH_3$ | 2 HCl | IP | -- | 80 | -- | -- | -- |
| $n-C_3H_7$ | $CH_3NH$ | H | HCl | IP | -- | 94 | 59 | 47 | -- |

**Table 2**

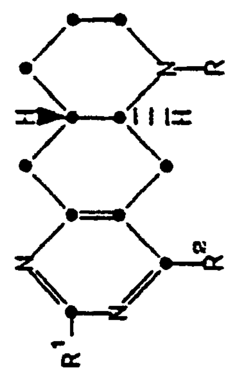

IIa

| R | $R^1$ | $R^2$ | Form | Route | 50 µg/kg | 10 µg/kg | 5 µg/kg | 1 µg/kg |
|---|---|---|---|---|---|---|---|---|
| $n-C_3H_7$ | $NH_2$ | H | 2 HCl | IP | 88 | 75 | 54 | 5 |
| $n-C_3H_7$ | $NH_2$ | H | 2 HCl | oral | 92 | 85 | 84 | 47 |

The compounds of formulae I and II are also active by the oral route. Trans-(±)-2-amino-6-n-propyl-5,5a,6,7,8,9,9a,10-octahydropyrimido[4,5-g]quinoline dihydrochloride, the second compound in Table 1, at 10 µg. by the oral route gave a 74% inhibition and 50 µg./kg. a 91% inhibition.

Compounds of formulae I and II, dopamine D-2 agonists, have been found to affect turning behavior in 6-hydroxydopamine-lesioned rats in a test procedure designed to uncover compounds useful for the treatment of Parkinsonism. In this test, nigroneostriatal-lesioned rats are employed, as prepared by the procedure of Ungerstedt and Arbuthnott, Brain Res, 24, 485 (1970). A compound having dopamine agonist activity causes the rats to turn in circles contralateral to the side of the lesion. After a latency period, which varies from compound to compound, the number of turns is counted over a 15-minute period.

Results obtained from such testing are set forth in Table 3 below. In the table, columns 1 and 2 give the substitution pattern for the compound at the head of the table, column 3, percent of test animals exhibiting turning behavior, and column 4, average number of turns observed in first 15 minutes after end of latency period.

Table 3

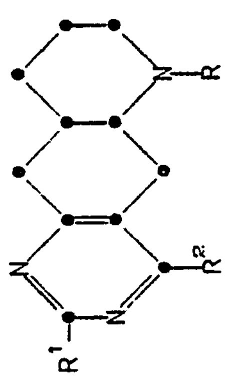

I

| R | $R^1$ | $R^2$ | % of rats exhibiting turning behavior--dose in mcg/kg 1000 | 100 | 20 | 10 | Average number of Turns per rat--dose in mcg/kg 1000 | 100 | 20 | 10 |
|---|---|---|---|---|---|---|---|---|---|---|
| $CH_3$ | $NH_2$ | H | 100 | -- | -- | -- | 38 | -- | -- | -- |
| $n-C_3H_7$ | $NH_2$ | H | 100 | -- | 94 | 50 | 62 | -- | 54 | 41 |
| $n-C_3H_7$ | $C_6H_5CO-NH$ | H | -- | 60 | -- | -- | -- | 51 | -- | -- |
| $n-C_3H_7$ | $CH_3CO-NH$ | H | 80 | -- | -- | -- | 42.5 | -- | -- | -- |
| $n-C_3H_7$ | $(CH_3)_2N$ | H | 100 | -- | -- | -- | 40.5 | -- | -- | -- |
| $n-C_3H_7$ | $NH_2$ | Cl | 83 | -- | -- | -- | 49 | -- | -- | -- |
| $n-C_3H_7$ | $NH_2$ | $CH_3$ | 100 | -- | -- | -- | 46 | -- | -- | -- |
| *$n-C_3H_7$ | $NH_2$ | H | -- | 100 | -- | -- | -- | 51 | -- | -- |
| $n-C_3H_7$ | $CH_3NH$ | H | 100 | -- | -- | -- | 33.3 | -- | -- | -- |

*5aR,9aR-isomer

The compounds of formula I and II are also active in affecting turning behavior by the oral route, although somewhat higher doses are required to give significant effects.

The compounds of formulae I and II reduce the blood pressure of spontaneously hypertensive rats, as illuminated by the following experiment:

Adult male spontaneously hypertensive rats (SHR) (Taconic Farms, Germantown, New York), weighing approximately 300 g. were anesthetized with pentobarbital sodium (60 mg./kg., i.p.). The trachea was cannulated and the SHR respired room air. Pulsatile arterial blood pressure was measured from a cannulated carotid artery using a Statham transducer (P23 ID). Mean arterial blood pressure was calculated as diastolic blood pressure plus 1/3 pulse pressure. Cardiac rate was monitored by a cardiotachometer

which was triggered by the systolic pressure pulse. Drug solutions were administered i.v. through a catheter placed in a femoral vein. Arterial blood pressure and cardiac rate were recorded on a multichannel oscillograph (Beckman, Model R511A). Fifteen minutes were allowed to elapse following surgery for equilibration of the preparation.

Table 4 which follows gives the results of this test for trans-(±)-2-amino-6-n-propyl-5,5a,6,7,8,9,9a,10-octahydropyrimido[4,5-g]quinoline. In Table 4, column 1 gives the dose level, column 2 the change in mean arterial blood pressure with standard error, and column 3, the percent change in cardiac rate with standard error.

## Table 4

| Dose level in mcg/kg | Percent Changes* | |
|---|---|---|
| | Mean Arterial Blood Pressure** | Cardiac Rate |
| 0.1 | -4.0±0.9 | -2.0±0.4 |
| 1 | -14.8±1.1 | -5.9±0.8 |
| 10 | -46.5±6.8 | -29.0±2.3 |
| 100 | -37.1±7.0 | -31.0±4.2 |

*Change was measured immediately after injection. Baseline mean arterial blood pressure was 181±1.0 mm Hg and mean cardiac rate was 366±15 beats/min.

**Mean response for 4SHR.

In addition, trans-(±)-2-amino-6-n-propyl-5,5a,6,7,8,9,9a,10-octahydropyrimido[4,5-g]quinoline and its trans-(-)-stereoisomer are potent activators of cholinergic neurons in rat striatum leading to an elevation of striatal acetyl choline concentrations.

The ability of a trans-(±) or trans-(-)-2-amino-4-permissibly-substituted-6-alkyl(or allyl)-octahydropyrimido[4,5-g]quinoline or a salt thereof (compounds of formulae I and II, respectively) to affect sexual behavior in male mammals is illustrated by the following experiment:

Male rats that required at least 5 minutes to achieve ejaculation were used. The behavioral tests were initiated with the introduction of a sexually receptive female rat into the behavioral arena and were stopped immediately following the first mount after ejaculation. The following behavioral indicies were measured:

| BEHAVIORAL INDEX | DEFINITION |
|---|---|
| 1. Mount Latency (ML): | Time from introduction of female to the first mount |
| 2. Intromission Latency (IL): | Time from introduction of female to the first intromission |
| 3. Ejaculatory Latency (EL): | Time interval from intromission to ejaculation |
| 4. Postejaculatory Interval (PEI): | Time interval from ejaculation to next mount |
| 5. Mount Frequency (MF): | Total number of mounts required to achieve ejaculation |
| 6. Intromission Frequency (IF): | Total number of mounts with intromission required to achieve ejaculation |

Each male rat was given a solution containing either the vehicle alone (1 millimolar acetic acid plus 1 millimolar ascorbic acid) in water or trans-(-)-2-amino-6-n-propyl-5,5a,6,7,8,9,9a,10-octahydropyrimido[4,5-g]quinolinedihydrochloride at 25 mcg./kg. in the same vehicle by subcutaneous injection 30 minutes prior to behavioral testing. One week after the drug test, the vehicle alone was retested.

The results of the above experiment are given in Table 5 below. In the Table, column 1 gives the treatment, and columns 2-7 the behavioral indices ($\bar{x}\pm$ SE for 9 rats) for each treatment in column 1.

Table 5

| Treatment | ML* | IL* | EL* | PEI* | MF** | IF** |
|---|---|---|---|---|---|---|
| Vehicle (Before) | 85.0 ±30.8 | 162.0 ±55.2 | 708.0 ±131.2 | 351.1 ±24.6 | 19.0 ±1.9 | 8.4 ±1.3 |
| Drug (25 mcg/kg s.c.) | 19.0 ±6.4 | 46.8 ±15.6 | 326.0 ±68.9 | 347.1 ±44.2 | 11.7 ±0.2 | 5.4 ±0.4 |
| Vehicle (After) | 122.6 ±56.6 | 144.8 ±62.5 | 640.0 ±63.0 | 343.4 ±21.1 | 21.1 ±3.7 | 5.7 ±0.9 |

*ML, IL, EL and PEI values are expressed in seconds.
**MF and IF values are expressed in number of mounts.

A repeat run at 0.25 mcg. was carried out with the following results (Table 6) (values represent $\bar{x}\pm$ SE for 11 rats).

Table 6

| Treatment | ML | IL | EL | PEI | MF | IF |
|---|---|---|---|---|---|---|
| Vehicle (Before) | 21.1 ±5.4 | 64.5 ±11.9 | 942.9 ±86.9 | 329.5 ±3.5 | 28.2 ±3.5 | 9.3 ±1.4 |
| Drug | 13.6 ±7.4 | 38.1 ±10.3 | 398.8 ±58.6 | 310.3 ±24.7 | 18.1 ±2.1 | 7.7 ±0.8 |
| Vehicle (After) | 12.4 +2.7 | 37.4 ±14.2 | 523.2 ±116.2 | 298.5 ±17.9 | 23.1 ±2.9 | 11.1 ±1.5 |

According to the data presented in Table 5, the drug produced statistically significant improvements in ejaculatory latency (EL) and mount frequency (MF) compared to either pre- or postcompound vehicle treatments and in intromission latency (IL) compared to prior vehicle treatment. These data are indicative of dramatic improvements in sexual performance specifically related to the effects of the drug. According to the data presented in Table 6, a single subcutaneous dose of drug of 250 ng/Kg produced statistically significant improvements in ejaculatory latency (EL) compared to prior vehicle treatment. Although there were no statistically significant differences in the comparison of the mean performance values between drug and subsequent vehicle responses, it is important to note that 6 of 11 rats showed better performance in each performance index following drug treating compared to the subsequent vehicle treatment, 9 and 8 of the 11 rats showed improvements in mount frequency and intromission frequency, respectively. These data are supportive of the view that trans-(-)-2-amino-6-n-propyl-5,5a,6,7,8,9,9a,10-octahydropyrimido[4,5-g]-quinoline dihydrochloride has behavioral effects in doses as low as 250 ng/Kg. Similar behavior experiments were conducted using drug doses of 2.5 ng/Kg, s.c., but no behavioral effects were observed.

The effects of trans-(-)-2-amino-6-n-propyl-5,5a,6,7,8,9,9a,10-octahydropyrimido[4,5-g]quinoline dihydrochloride on male rat sexual behavior were also evaluated in rats that showed no mating behavior or were unable to achieve ejaculation in the 30 minute test period. The effects of 25 mcg/Kg, s.c. of drug on the mating performance of these animals are summarized in Table 7. The drug appeared to have the

capacity to initiate sexual behavior in animals that showed no prior sexual behavior and to amplify sexual behavior in animals that were unable to achieve ejaculation. The mating performance of rats from these groups that were able to achieve ejaculation after drug treatment and subsequent vehicle treatments were evaluated. These animals showed a significant reduction in the number of mounts required for ejaculation (mount frequency) with drug treatment compared to vehicle treatment.

**Table 7**

EFFECT OF A SINGLE ADMINISTRATION OF DRUG (25 MCG/KG, S.C..) ON MATING PERFORMANCE OF IMPOTENT RATS

| PERCENTAGE OF RATS DISPLAYING MATING BEHAVIOR | |
|---|---|
| Vehicle (1 week prior) | 0.0% (0/8) |
| Drug | 87.5% (7/8) |
| Vehicle (1 week after) | 37.5% (3/8) |

| PERCENTAGE OF RATS ABLE TO ACHIEVE EJACULATION IN 30 MIN. | |
|---|---|
| Vehicle (before) | 0.0% (0/14) |
| Drug | 92.9% (13/14) |
| Vehicle (after) | 50.0% (7/14) |

ACTIVITY OF MALES ACHIEVING EJACULATION

| | ML | IL | EL | PEI | IF | MF |
|---|---|---|---|---|---|---|
| Drug (N=7) | 34.4 ±13.7 | 79.6 ±42.3 | 298.4 ±48.5 | 308.1 ±36.9 | 4.9 ±1.0 | 8.7 ±1.0 |
| Post Vehicle (N=7) | 50.3 ±22.0 | 80.8 ±31.6 | 537.1 ±110.5 | 291.3 ±26.1 | 6.1 ±1.1 | 27.1* ±3.9 |

*Significantly greater than drug (P <.003)

The effects of trans-(±)- and trans-(-)-2-amino-6-n-propyl-5,5a,6,7,8,9,9a,10-octahydropyrimido[4,5-g]-quinoline dihydrochloride on sexual behavior of female mammals were evaluated in ovariectomized, estrogen-treated rats. The change in the lordosis-to-mount ratio was measured (increase in presenting by the female for mounting by a male per mount). The protocol of Foreman and Moss, Physiology and Behavior, 22, 283 (1979), was used. Table 8 which follows gives the results of this experiment. In the Table, column 1 gives the name of the drug used, if any, column 2 the dose in mcg./kg. and column 3 the change in lordosis-to-mount ratio with standard error.

Table 8

| Treatment | Dose (s.c.) (mcg./kg.) | Change in Lordosis-to-Mount Ratio ($\bar{x}$ ± SE) |
|---|---|---|
| Vehicle | | .158 ± .042 |
| Trans-(±)-Racemate | 25 | .580 ± .063* |
| Trans-(-)-Stereoisomer | 25 | .760 ± .058** |

* Significantly greater than vehicle P<.05
**Significantly greater than trans-(±) P<.05

A similar experiment was carried out on the two stereoisomers; trans-(-)-2-amino-6-n-propyl-5,5a,6,7,8,9,9a,10-octahydropyrimido[4,5-g]quinoline and the trans-(+)-isomer. Response to the trans-(+)-isomer was not significantly greater than the response to vehicle alone (.093 ± .063 to .035 ± .018) whereas the trans-(-)-isomer gave a highly significant change .753 ± .031.

Table 9 below gives the effect of a series of dose levels of trans-(-)-2-amino-6-n-propyl-5,5a,6,7,8,9,9a,10-octahydropyrimido[4,5-g]quinoline on the lordosis-to-mount ratio in ovariectomized, estrogen-treated rats.

Table 9

| Dosage mcg./kg. | Route | Change In Lordosis-To-Mount Ratio** |
|---|---|---|
| 0 | SC | 0.074 ± 0.025 |
| 2.5 | SC | 0.284 ± 0.064 |
| 7.5 | SC | 0.405 ± 0.083 |
| 25 | SC | 0.786 ± 0.028 |
| 0 | oral | 0.008 ± 0.021 |
| 2.5 | oral | 0.467 ± 0.033 |
| 25 | oral | 0.558 ± 0.063 |

**All values are $\bar{X}$ ± SE for 19 animals (S.C.) and for 8 animals oral (water as the vehicle).

Behavioral response to vehicle was significantly lower (P<.01) than response to drug at each dosage and route of administration.

The compounds of formula IIa, the trans-(+)-stereoisomers or 5aS,9aS-stereoisomers, are dopamine D-1 agonists. Compounds represented by the formula IIa manifest their dopamine D-1 agonist activity in several ways; for example, one way is in stimulating cyclic AMP formation in rat striatal membrane.

In this determination, the procedure of Wong and Reid, Communications in Psychopharmacology, 4, 269 (1980) was employed. 5a5,9aS-2-amino-6-n-propyl-5,5a,6,7,8,9,9a,10-octahydropyrimido[4,5-g]quinoline (formula IIa), the 5aR,9aR enantiomer (formula II), and the corresponding racemate (formula I) were tested

for their ability to activate adenylate cyclase in rat striatal membrane as measured by an increase in cyclic AMP concentration. The results of this determination are given in Table 10. Dopamine was used as a positive control. In the table, column 1 gives the drug used, column 2, drug concentration in the reaction mixture, column 3, cyclic AMP formation as percent of control, and column 4, confidence level or significance.

Table 10

| Drug | Conc. in micromoles | Cyclic AMP Formation as a percent of control | P |
|---|---|---|---|
| Dopamine | 100 | 160.0 ± 14.8 | <0.01 |
| Racemate | 1 | 130.8 ± 0.8 | <0.05 |
| 5aR,9aR enantiomer | 1 | 119.2 ± 1.3 | <0.001 |
| 5aS,9aS enantiomer | 1 | 165.0 ± 3.1 | <0.01 |

In the presence of 10 micromolar GTP, the basal adenylate cyclase activity in rat striatal membranes has a mean ± S.E. value of 196.2 ± 20.3 pmole/min/mg of protein. Compounds were examined in triplicate.

According to the information presented in Table 10, the 5aS,9aS enantiomer of formula IIa significantly increased cyclic AMP formation, indicating significant D-1 dopamine agonist activity. It was more effective than the racemate while the increase of cyclic AMP induced by the 4aR,9aR enantiomer of formula II barely met the required significance level.

A second, particularly sensitive indicator of D-1 agonist activity is the determination of the cyclic AMP efflux in tissue slices using a procedure based on Stoof and Kebabian, Nature, 294, 266 (1981) and Brain Res., 250, 263 (1982). In this procedure, striatal tissue is dissected from rat brains and chopped into 0.3 mm. x .03 mm. fragments. The tissue fragments are suspended in the appropriate buffer system (Earl's balanced salt solution, for example) and the suspension is continuously aerated with 95:5 $O_2/CO_2$ while being maintained at 37° C. Just prior to use, the tissue fragments are transferred to fresh media to which is added bovine serum albumin (2.5 mg./ml.) and 3-isobutyl-1-methylxanthine (1 millimolar) to block degradation of cyclic AMP. The tissue fragments are incubated in buffer without drugs and are then transferred to the same media with added drug. Aliquots of incubation media, with and without drugs, are assayed for cyclic AMP concentration by a specific radioimmunoassay. The effect of the drug or drugs on efflux is expressed as a percentage of the resting efflux. The following medium was employed:

| Medium Ingredient | Conc. mg./Liter |
|---|---|
| NaCl | 6800 |
| KCl | 402.6 |
| $NaHCO_3$ | 2201.1 |
| $NaH_2PO_4$ | 137.99 |
| $MgSO_4 \cdot 7H_2O$ | 147.88 |
| d-glucose | 1009 |
| $CaCl_2 \cdot 2H_2O$ | 191.1 |
| phenol red | 10 |

In the experiments described in the above-mentioned reference, Stoof and Kebabian employed sulpiride to repress the negative (anti D-1) effect of any drug acting as a dopamine D-2 agonist. The authors had previously demonstrated that a D-2 agonist repressed cyclic AMP formation (an opposite effect from that produced by a D-1 agonist). Sulpiride is known to be an antagonist on pituitary D-2 receptors. Addition of sulpiride to a test system, such as that described above, blocks any D-2 effect of a drug in reducing cyclic

AMP production. Stoof and Kebabian were able to demonstrate a lack of effect of sulpiride on cyclic AMP production in striatal tissue using SKF 38393 (1,2,3,4-tetrahydro-7,8-dihydroxy-1-phenyl-1H-3-benzazepine) as a pure D-1 agonist, an indication that D-2 receptors were not involved and the compound did not have D-2 agonist activity.

The results of one such determination for 5aS,9aS-2-amino-6-n-propyl-5,5a,6,7,8,9,9a,10-octahydropyrimido[4,5-g]quinolinedihydrochloride (compound A) are given in Table 11. In the table, column 1 gives the added drug, if any; column 2, the drug concentration; column 3, the level of cyclic AMP present; and column 4, the percent increase in cyclic AMP concentration.

Table 11

| Drug | Concentration | Cyclic AMP Concentration | % Increase in Cyclic AMP |
|---|---|---|---|
| None | | 71.6 ± 8.2 | 0 |
| A | $5 \times 10^{-6}$M | 114.0 ± 11.2 | 60 |

The compounds of formula I, II or IIa are administered for therapeutic purposes in a variety of formulations as illustrated below. However, it should be remembered that the dosage amounts of the trans-(±)-and (5aR,9aR)-stereoisomers, the dopamine D-2 agonists of formulae I and II, are far less than the dosage amounts of the (5aS,9aS)-stereoisomers, the dopamine D-1 agonists of formula IIa.

Hard gelatin capsules are prepared using the following ingredients:

| | Quantity (mg./capsule) |
|---|---|
| Active compound | .1-2 mg |
| Starch dried | 200 |
| Magnesium stearate | 10 |

The above ingredients are mixed and filled into hard gelatin capsules.

A tablet formulation is prepared using the ingredients below:

| | Quantity (mg./tablet) |
|---|---|
| Active compound | .1-2 mg |
| Cellulose, microcrystalline | 400 |
| Silicon dioxide, fumed | 10 |
| Stearic acid | 5 |

The components are blended and compressed to form tablets.

Alternatively, tablets each containing .1-2 mg. of active ingredient are made up as follows:

| | |
|---|---|
| Active ingredient | .1-2 mg. |
| Starch | 45 mg. |
| Microcrystalline cellulose | 35 mg. |
| Polyvinylpyrrolidone (as 10% solution in water) | 4 mg. |
| Sodium carboxymethyl starch | 4.5 mg. |
| Magnesium stearate | 0.5 mg. |
| Talc | 1 mg. |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60° C. and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets.

Capsules each containing 0.1-2 mg. of medicament are made as follows:

| | |
|---|---|
| Active ingredient | .1-2 mg. |
| Starch | 59 mg. |
| Microcrystalline cellulose | 59 mg. |
| Magnesium stearate | 2 mg. |

The active ingredient, cellulose, starch and magnesium stearate are blended, passed through a No. 45 mesh U.S. sieve, and filled into hard gelatin capsules.

Suspensions each containing .1-2 mg. of medicament per 5 ml. dose are made as follows:

| | |
|---|---|
| Active ingredient | .1-2 mg. |
| Sodium carboxymethyl cellulose | 50 mg. |
| Syrup | 1.25 ml. |
| Benzoic acid solution | 0.10 ml. |
| Flavor | q.v. |
| Color | q.v. |
| Purified water to | 5 ml. |

The medicament is passed through a No. 45 mesh U.S. sieve and mixed with the sodium carboxymethylcellulose and syrup to form a smooth paste. The benzoic acid solution, flavor and color are diluted with some of the water and added, with stirring. Sufficient water is then added to produce the required volume.

For oral administration, tablets, capsules or suspensions containing from about .1 to about 2 mg. of an active D-2 agonist per dose are given 3-4 times a day, giving a daily dosage of .3 to 8 mgs. or, for a 75 kg person, about 4.0 to about 107 mcg/kg. The intravenous dose is in the range from about .1 to about 100 mcg./kg.

For the D-1 agonists, the dose level sufficient to stimulate D-1 receptors varies from .01-15 mg./kg./day depending on the active compound employed. The above formulations using .01-15 mg. of active ingredient are employed.

## Claims

1. A compound of the formula

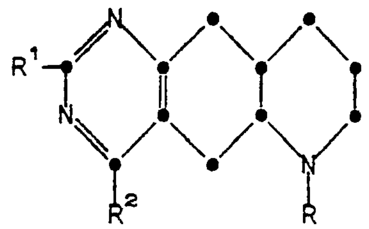

I

wherein
R is $C_1$-$C_3$ alkyl or allyl;
$R^2$ is H, $CH_3$, Cl or Br;
$R^1$ is $NH_2$, $NHR^3$ or $NR^4R^5$;
wherein $R^3$ is methyl, ethyl, n-propyl or $R^6$-CO where $R^6$ is $C_1$-$C_3$ alkyl or

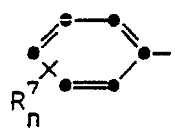

where $R^7$ is independently H, Cl, F, Br, $CH_3$, $C_2H_5$, $CH_3O$, $C_2H_5O$ or $CF_3$; and n is 0, 1 or 2;
wherein $R^4$ and $R^5$ are independently methyl, ethyl or n-propyl; or
a pharmaceutically-acceptable acid addition salt thereof.

2. A compound of claim 1 being the trans-(-)-stereoisomer of the formula

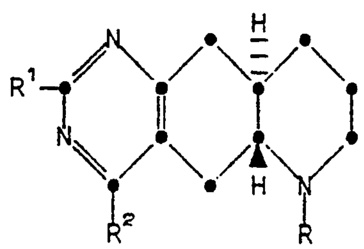

II

wherein R, $R^1$ and $R^2$ are defined as in claim 1, or a pharmaceutically-acceptable acid addition salt thereof.

3. A compound of claim 1 being the trans-(+)-stereoisomer of the formula

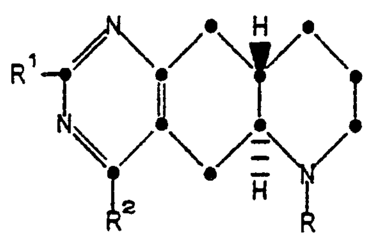

IIa

wherein R, $R^1$ and $R^2$ are defined as in claim 1, or a pharmaceutically-acceptable acid addition salt thereof.

4. A compound of claim 1, 2 or 3 of formula I, II or IIa in which R is n-propyl.

5. Any one of the following compounds, or a pharmaceutically-acceptable acid addition salt thereof:
trans-(±)-2-amino-6-methyl-5,5a,6,7,8,9,9a,10-octahydropyrimido[4,5-g]quinoline
trans-(±)-2-amino-6-n-propyl-5,5a,6,7,8,9,9a,10-octahydropyrimido[4,5-g]quinoline
5aR,9aR-2-amino-6-n-propyl-5,5a,6,7,8,9,9a,10-octahydropyrimido[4,5-g]quinoline
trans-(±)-2-dimethylamino-6-n-propyl-5,5a,6,7,8,9,9a,10-octahydropyrimido[4,5-g]quinoline
trans-(±)-2-methylamino-6-n-propyl-5,5a,6,7,8,9,9a,10-octahydropyrimido[4,5-g]quinoline
trans-(±)-2-amino-4-methyl-6-n-propyl-5,5a,6,7,8,9,9a,10-octahydropyrimido[4,5-g]quinoline
trans-(±)-2-amino-4-chloro-6-n-propyl-5,5a,6,7,8,9,9a,10-octahydropyrimido[4,5-g]quinoline
trans-(±)-2-acetylamino-6-n-propyl-5,5a,6,7,8,9,9a,10-octahydropyrimido[4,5-g]quinoline
trans-(±)-2-benzoylamino-6-n-propyl-5,5a,6,7,8,9,9a,10-octahydropyrimido[4,5-g]quinoline
5aS,9aS-2-amino-6-n-propyl-5,5a,6,7,8,9,9a,10-octahydropyrimido[4,5-g]quinoline.

6. A process for preparing a compound of formula I, II or IIa, or a pharmaceutically-acceptable acid addition salt thereof, whenever as claimed in any one of claims 1 to 5, which comprises:
(a) condensing a compound of the formula

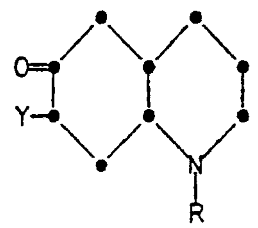

V

wherein R is as defined as above;
Y is $CH_3CO$, or $(R^{10})_2NCH=$, wherein each $R^{10}$ is independently $C_1$-$C_3$ alkyl, or one $R^{10}$ is H and the other is $C_1$-$C_3$ alkyl;
with a compound or its salt of the formula

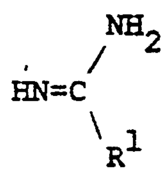

VI

wherein $R^1$ is defined as above to provide a compound of formula I; or

(b) alkylating a compound of the formula

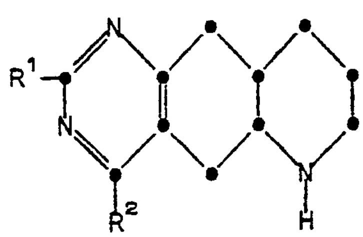

Ia

wherein $R^1$ and $R^2$ are defined as above to provide the compounds of formula I; or
(c) halogenating a compound of the formula

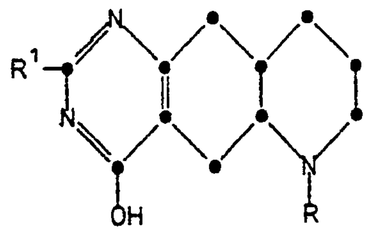

If

wherein R and $R^1$ are defined as above to provide the compounds of formula I where $R^2$ is Cl or Br; or

(d) acylating a compound of formula I wherein $R^1$ is $NH_2$ to provide a compound of formula I in which $R^1$ is $NHR^3$ where $R^3$ is $R^6$-CO; and

(e) optionally followed by salifying to form the pharmaceutically-acceptable acid addition salt of the product of formula I and/or resolving a racemic product to form the optically active product of formula I.

**7.** A pharmaceutical formulation which comprises as an active ingredient a compound of formula I, II or IIa, or a pharmaceutically-acceptable acid addition salt thereof, as claimed in any one of claims 1 to 5, associated with one or more pharmaceutically-acceptable carriers or diluents therefor.

**8.** A pharmaceutical formulation of claim 7 adapted for oral or IP administration.

**9.** A compound of formula I or II, or a pharmaceutically-acceptable acid addition salt thereof, as claimed in claim 1, 2, 4 or 5, for use as a D-2 dopamine agonist.

**10.** A compound of formula I or IIa, or a pharmaceutically-acceptable acid addition salt thereof, as claimed in claim 1, 3, 4 or 5, for use as a D-1 dopamine agonist.

**11.** A compound of the formula

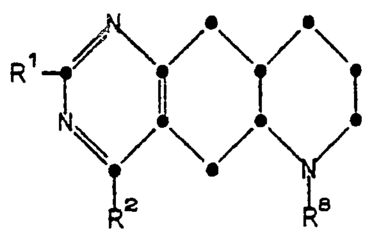

III

wherein $R^1$ and $R^2$ are defined as in claim 1, and $R^8$ is H, CN or ($C_1$-$C_3$ alkoxy)-CO; or an acid addition salt thereof.

**12.** A compound of claim 11 being the trans-(-)-stereoisomer of the formula

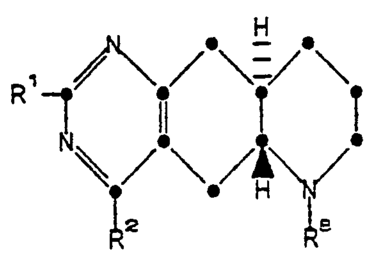

IV

wherein $R^1$, $R^2$ and $R^8$ are defined as in claim 11; or an acid addition salt thereof.

**13.** A compound of claim 11 being the trans-(+)-stereoisomer of the formula

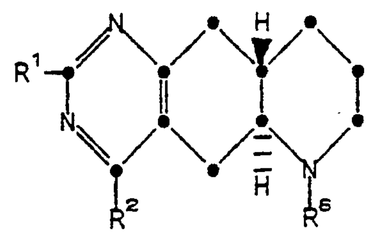

IVa

wherein $R^1$ R2 and $R^8$ are defined as in claim 11; or an acid addition salt thereof.

**14.** A trans-(±) compound of the formula

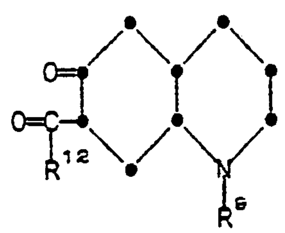

Va

where $R^9$ is $C_1$-$C_3$ alkyl, CN, H or allyl; and $R^{12}$ is methyl or $C_1$-$C_3$ alkyloxy; or an acid addition salt thereof.

**15.** A trans-(-)-stereoisomer of a compound of claim 14.

**16.** A trans-(+)-stereoisomer of a compound of claim 14.

**17.** Trans-(-)-2-amino-6-n-propyl-5,5a,6,7,8,9,9a,10-octahydropyrimidoquinoline or a pharmaceutically-acceptable salt thereof.

**18.** Trans-(-)-2-amino-6-n-propyl-5,5a,6,7,8,9,9a,10-octahydropyrimidoquinoline dihydrochloride.

Claims for the following Contracting State: AT

**1.** A process for preparing a compound of the formula

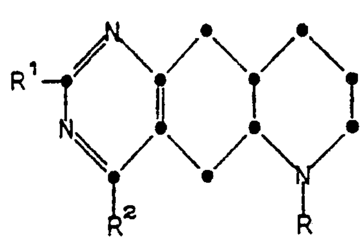

I

wherein
R is $C_1$-$C_3$ alkyl or allyl;
$R^2$ is H, $CH_3$, Cl or Br;
$R^1$ is $NH_2$, $NHR^3$ or $NR^4R^5$;
wherein $R^3$ is methyl, ethyl, n-propyl or $R^6$-CO where $R^6$ is $C_1$-$C_3$ alkyl or

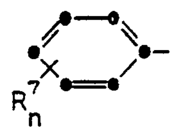

where $R^7$ is independently H, Cl, F, Br, $CH_3$, $C_2H_5$, $CH_3O$, $C_2H_5O$ or $CF_3$; and n is 0, 1 or 2; wherein $R^4$ and $R^5$ are independently methyl, ethyl or n-propyl; or a pharmaceutically-acceptable acid addition salt thereof; which comprises:

(a) condensing a compound of the formula

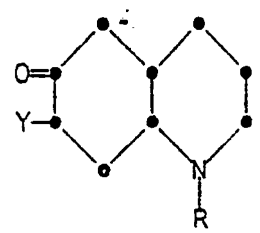

V

wherein R is as defined as above;
Y is $CH_3CO$, or $(R^{10})_2NCH=$, wherein each $R^{10}$ is independently $C_1$-$C_3$ alkyl, or one $R^{10}$ is H and the other is $C_1$-$C_3$ alkyl;
with a compound or its salt of the formula

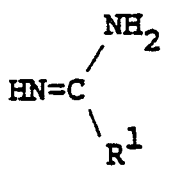

VI

wherein $R^1$ is defined as above to provide a compound of formula I; or
(b) alkylating a compound of the formula

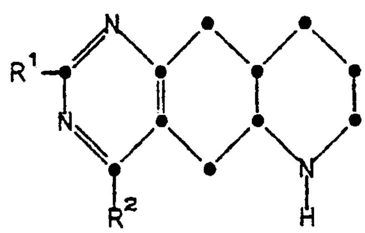

Ia

wherein $R^1$ and $R^2$ are defined as above to provide the compounds of formula I; or
(c) halogenating a compound of the formula

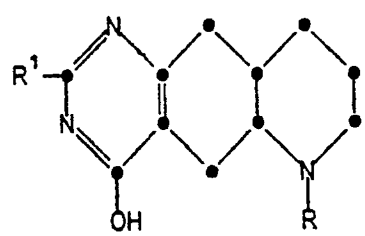

If

wherein R and $R^1$ are defined as above to provide the compounds of formula I where $R^2$ is Cl or Br; or

(d) acylating a compound of formula I wherein $R^1$ is $NH_2$ to provide a compound of formula I in which $R^1$ is $NHR^3$ where $R^3$ is $R^6$-CO; and
(e) optionally followed by salifying to form the pharmaceutically-acceptable acid addition salt of the product of formula I and/or resolving a racemic product to form the optically active product of formula I.

2. A process of claim 1 for preparing the trans-(-)-stereoisomer of formula I.

3. A process of claim 1 or 2 for preparing a compound of formula I wherein R is n-propyl, $R^1$ is amino, and $R^2$ is hydrogen.

4. The process of claim 1 for preparing trans-(±)-2-amino-6-methyl-5,5a,6,7,8,9,9a,10-octahydropyrimido-[4,5-g]quinoline which comprises reacting trans-(±)-1-methyl-6-oxo-7-(dimethylaminomethylene)-decahydroquinoline with guanidine carbonate.

5. The process of claim 1 for preparing traps-(±)-2-amino-6-n-propyl-5,5a,6,7,8,9,9a,10-octahydropyrimido-[4,5-g]quinoline which comprises reacting trans-(±)-1-n-propyl-6-oxo-7-(dimethylaminomethylene)-decahydroquinoline with guanidine carbonate.

6. The process of claim 2 for preparing 5aR,9aR-2-amino-6-n-propyl-5,5a,6,7,8,9,9a,10-octahydropyrimido-[4,5-g]quinoline which comprises reacting 4aR,8aR-1-n-propyl-6-oxo-7-(dimethylaminomethylene)-decahydroquinoline with guanidine carbonate.

7. The process of claim 1 for preparing trans-(±)-2-dimethylamino-6-n-propyl-5,5a,6,7,8,9,9a,10-octahydropyrimido[4,5-g]quinoline which comprises reacting trans-(±)-1-n-propyl-6-oxo-7-(dimethylaminomethylene)decahydroquinoline with N,N-dimethylguanidine hydrochloride.

8. The process of claim 1 for preparing trans-(±)-2-methylamino-6-n-propyl-5,5a,6,7,8,9,9a,10-octahydropyrimido[4,5-g]quinoline which comprises reacting trans-(±)-1-n-propyl-6-oxo-7-(dimethylaminomethylene)decahydroquinoline with N-methylguanidine.

9. The process of claim 1 for preparing trans-(±)-2-amino-4-methyl-6-n-propyl-5,5a,6,7,8,9,9a,10-octahydropyrimido[4,5-g]quinoline which comprises reacting trans-(±)-1-n-propyl-6-oxo-7-acetyl-decahydroquinoline with guanidine carbonate.

10. The process of claim 1 for preparing trans-(±)-2-amino-4-chloro-6-n-propyl-5,5a,6,7,8,9,9a,10-octahydropyrimido[4,5-g]quinoline which comprises reacting trans-(±)-2-amino-4-hydroxy-6-n-propyl-5,5a,6,7,8,9,9a,10-octahydropyrimido[4,5-g]quinoline with phosphorous oxychloride.

11. The process of claim 1 for preparing trans-(±)-2-acetylamino-6-n-propyl-5,5a,6,7,8,9,9a,10-octahydropyrimido[4,5-g]quinoline which comprises reacting trans-(±)-2-amino-6-n-propyl-5,5a,6,7,8,9,9a,10-octahydropyrimido[4,5-g]quinoline with acetic anhydride.

12. The process of claim 1 for preparing trans-(±)-2-benzoylamino-6-n-propyl-5,5a,6,7,8,9,9a,10-octahydropyrimido[4,5-g]quinoline which comprises reactingtrans-(±)-2-amino-6-n-propyl-5,5a,6,7,8,9,9a,10-octahydropyrimido[4,5-g]quinoline with benzoyl chloride.

13. The process of claim 1 for preparing 5aS,9aS-2-amino-6-n-propyl-5,5a,6,7,8,9,9a,10-octahydropyrimido-[4,5-g]quinoline which comprises reacting 4aS,8aS-1-n-propyl-6-oxo-7-(dimethylaminomethylene)-decahydroquinoline with guanidine carbonate.

14. The process of claim 1 for preparing trans-(-)-2-amino-6-n-propyl-5,5a,6,7,8,9,9a,10-octahydropyrimidoquinoline or a pharmaceutically-acceptable salt thereof.

15. The process of claim 1 for preparing trans-(-)-2-amino-6-n-propyl-5,5a,6,7,8,9,9a,10-octahydropyrimidoquinoline dihydrochloride.

## Revendications

1. Composé de formule

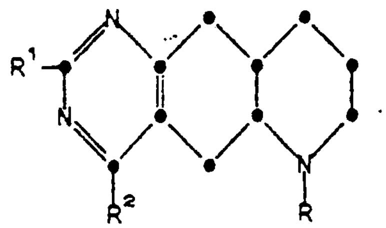

I

dans laquelle :
R représente un groupe alkyle en $C_1$-$C_3$ ou un groupe allyle ;
$R^2$ représente H, $CH_3$, Cl ou Br ;
$R^1$ représente $NH_2$, $NHR^3$ ou $NR^4R^5$ ;
où $R^3$ représente un groupe méthyle, un groupe éthyle, un groupe n-propyle ou $R^6$-CO, où $R^6$ représente un groupe alkyle en $C_1$-$C_3$ ou

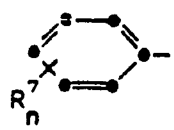

où $R^7$ représente, de manière indépendante, H, Cl, F, Br, $CH_3$, $C_2H_5$, $CH_3O$, $C_2H_5O$ ou $CF_3$ ; et n est égal à 0, 1 ou 2 ;
où $R^4$ et $R^5$ représentent, indépendamment l'un de l'autre, un groupe méthyle, un groupe éthyle ou un groupe n-propyle ; ou
un de ses sels d'addition d'acides pharmaceutiquement acceptables.

2. Composé de formule 1, à savoir le stéréo-isomère trans-(-) de formule

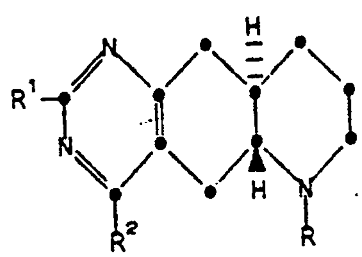

II

dans laquelle R, $R^1$ et $R^2$ sont tels que définis dans la revendication 1 ou un de ses sels d'addition d'acides pharmaceutiquement acceptables.

3. Composé de formule 1, à savoir le stéréo-isomère trans-(+) de formule

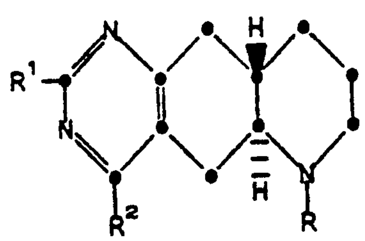

IIa

dans laquelle R, $R^1$ et $R^2$ sont tels que définis dans la revendication 1 ou un de ses sels d'addition d'acides pharmaceutiquement acceptables.

4. Composé selon la revendication 1, 2 ou 3 de formule I, II ou IIa, dans laquelle R représente un groupe n-propyle.

5. L'un quelconque des composés ci-après ou un de leurs sels d'addition d'acides pharmaceutiquement acceptables :
la trans-(±)-2-amino-6-méthyl-5,5a,6,7,8,9,9a,10-octahydropyrimido[4,5-g]quinoléine ;
la trans-(±)-2-amino-6-n-propyl-5,5a,6,7,8,9,9a,10-octahydropyrimido[4,5-g]quinoléine ;
la 5aR,9aR-2-amino-6-n-propyl-5,5a,6,7,8,9,9a,10-octahydropyrimido[4,5-g]quinoléine ;
la trans-(±)-2-diméthylamino-6-n-propyl-5,5a,6,7,8,9,9a,10-octahydropyrimido[4,5-g]quinoléine ;
la trans-(±)-2-méthylamino-6-n-propyl-5,5a,6,7,8,9,9a,10-octahydropyrimido[4,5-g]quinoléine ;
la trans-(±)-2-amino-4-méthyl-6-n-propyl-5,5a,6,7,8,9,9a,10-octahydropyrimido[4,5-g]quinoléine ;
la trans-(±)-2-amino-4-chloro-6-n-propyl-5,5a,6,7,8,9,9a,10-octahydropyrimido[4,5-g]quinoléine ;
la trans-(±)-2-acétylamino-6-n-propyl-5,5a,6,7,8,9,9a,10-octahydropyrimido[4,5-g]quinoléine ;
la trans-(±)-2-benzoylamino-6-n-propyl-5,5a,6,7,8,9,9a,10-octahydropyrimido[4,5-g]quinoléine ;
la 5aS,9aS-2-amino-6-n-propyl-5,5a,6,7,8,9,9a,10-octahydropyrimido[4,5-g]quinoléine.

6. Procédé pour la préparation de l'un quelconque des composés de formule I, II ou IIa ou d'un de leurs sels d'addition d'acides pharmaceutiquement acceptables, tels que revendiqués dans l'une quelconque des revendications 1 à 5, ce procédé consistant à :
(a) condenser un composé de formule

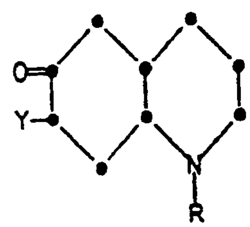

V

dans laquelle R est tel que défini ci-dessus ;
Y représente $CH_3CO$ ou $(R^{10})_2NCH=$, où chacun des radicaux $R^{10}$ représente, indépendamment l'un de l'autre, un groupe alkyle en $C_1$-$C_3$, ou encore un des radicaux $R^{10}$ représente H et l'autre représente un groupe alkyle en $C_1$-$C_3$ ;
avec un composé ou son sel de formule

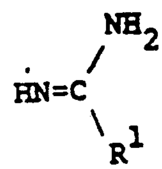

VI

dans laquelle $R^1$ est défini tel que ci-dessus, pour obtenir un composé de formule I ; ou

(b) alkyler un composé de formule

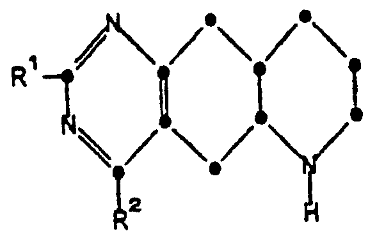

Ia

dans laquelle $R^1$ et $R^2$ sont tels que définis ci-dessus pour obtenir les composés de formule I ; ou
(c) halogéner un composé de formule

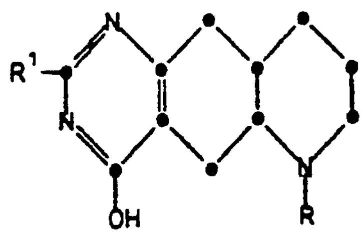

If

dans laquelle R et $R^1$ sont tels que définis ci-dessus pour obtenir les composés de formule I dans laquelle $R^2$ représente Cl ou Br ; ou
(d) acyler un composé de formule I dans laquelle $R^1$ représente $NH_2$ pour obtenir un composé de formule I dans laquelle $R^1$ représente $NHR^3$ où $R^3$ représente $R^6$-CO ; et
(e) éventuellement faire suivre d'une salification pour obtenir le sel d'addition d'acide pharmaceutiquement acceptables du produit de formule I et/ou du dédoublement d'un produit racémique pour obtenir le produit optiquement actif de formule I.

**7.** Formualtion pharmaceutique qui comprend, comme ingrédient actif, un composé de formule I, II ou IIa, ou un de ses sels d'addition d'acides pharmaceutiquement acceptables, tel que revendiqué dans l'une quelconque des revendications 1 à 5, en association avec un ou plusieurs supports ou diluants pour ce composé.

**8.** Formulation pharmaceutique selon la revendication 7 conçue pour l'administration par voie orale ou par voie intrapéritonéale.

**9.** Composé de formule I ou II, ou un de ses sels d'addition d'acides pharmaceutiquement acceptables tel que revendiqué dans la revendication 1, 2, 4 ou 5, pour être utilisé comme agoniste D-2 de la dopamine.

**10.** Composé de formule I ou IIa, ou un de ses sels d'addition d'acides pharmaceutiquement acceptables tel que revendiqué dans la revendication 1, 3, 4 ou 5, pour être utilisé comme agoniste D-1 de la dopamine.

**11.** Composé de formule :

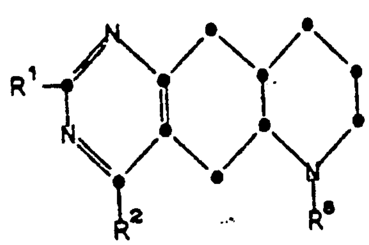

III

dans laquelle $R^1$ et $R^2$ sont tels que définis dans la revendication 1 et $R^8$ représente H, CN ou un groupe (alcoxy en $C_1$-$C_3$)-CO, ou un de ses sels d'addition d'acides.

**12.** Composé selon la revendication 11, à savoir le stéréo-isomère trans-(-) de formule

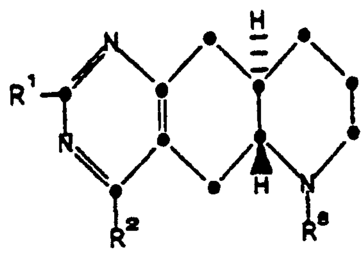

IV

dans laquelle $R^1$, $R^2$ et $R^8$ sont tels que définis dans la revendication 11, ou un de ses sels d'addition d'acides.

**13.** Composé selon la revendication 11, à savoir le stéréo-isomère trans-(+) de formule

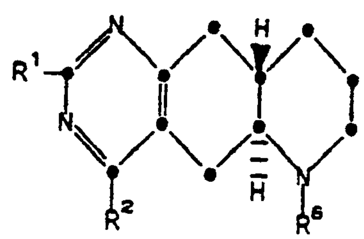

IVa

dans laquelle $R^1$, $R^2$ et $R^8$ sont tels que définis dans la revendication 11, ou un de ses sels d'addition d'acides.

**14.** Composé trans-(±) de formule

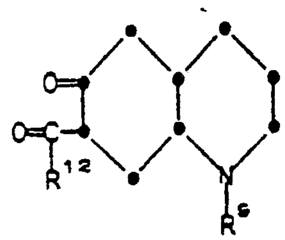

Va

dans laquelle $R^9$ représente un groupe alkyle en $C_1$-$C_3$, CN, H ou un groupe allyle ; et $R^{12}$ représente un groupe méthyle ou un groupe alkyl(en $C_1$-$C_3$)oxy ; ou un de ses sels d'addition d'acides.

**15.** Stéréo-isomère trans-(-) d'un composé de formule 14.

**16.** Stéréo-isomère trans-(+) d'un composé de formule 14.

**17.** Trans-(-)-2-amino-6-n-propyl-5,5a,6,7,8,9,9a,10-octahydropyrimidoquinoléine ou un de ses sels d'addition d'acides pharmaceutiquement acceptables.

**18.** Dichlorhydrate de la trans-(-)-2-amino-6-n-propyl-5,5a,6,7,8,9,9a,10-octahydropyrimidoquinoléine.

Revendications pour l'Etat contractant: AT

**1.** Procédé pour préparer un composé de formule

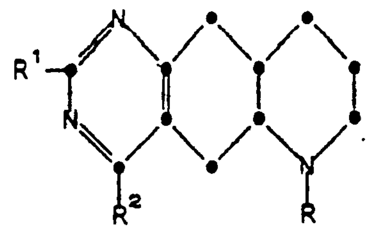

I

dans laquelle :
R représente un groupe alkyle en $C_1$-$C_3$ ou un groupe allyle ;
$R^2$ représente H, $CH_3$, Cl ou Br ;
$R^1$ représente $NH_2$, $NHR^3$ ou $NR^4R^5$ ;
où $R^3$ représente un groupe méthyle, un groupe éthyle, un groupe n-propyle ou $R^6$-CO, où $R^6$ représente un groupe alkyle en $C_1$-$C_3$ ou

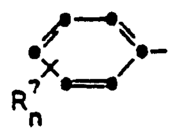

où $R^7$ représente, de manière indépendante, H, Cl, F, Br, $CH_3$, $C_2H_5$, $CH_3O$, $C_2H_5O$ ou $CF_3$ ; et n est égal à 0, 1 ou 2 ;
où $R^4$ et $R^5$ représentent, indépendamment l'un de l'autre, un groupe méthyle, un groupe éthyle ou un groupe n-propyle ; ou
un de ses sels d'addition d'acides pharmaceutiquement acceptables ;
ce procédé consistant à :

(a) condenser un composé de formule

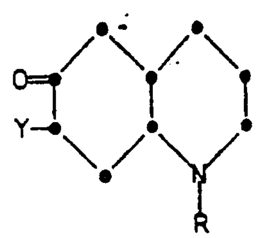

V

dans laquelle R est tel que défini ci-dessus ;
Y représente $CH_3CO$ ou $(R^{10})_2NCH=$, où chacun des radicaux $R^{10}$ représente, indépendamment l'un de l'autre, un groupe alkyle en $C_1$-$C_3$, ou encore un des radicaux $R^{10}$ représente H et l'autre représente un groupe alkyle en $C_1$-$C_3$ ;
avec un composé ou son sel de formule

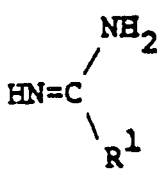

VI

dans laquelle $R^1$ est défini tel que ci-dessus, pour obtenir un composé de formule I ; ou
(b) alkyler un composé de formule

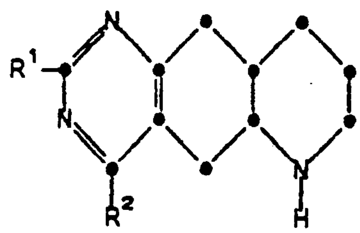

Ia

dans laquelle $R^1$ et $R^2$ sont tels que définis ci-dessus pour obtenir les composés de formule I ; ou

(c) halogéner un composé de formule

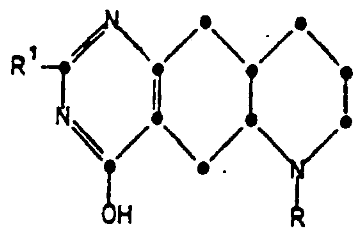

If

dans laquelle R et $R^1$ sont tels que définis ci-dessus pour obtenir les composés de formule I dans laquelle $R^2$ représente Cl ou Br ; ou
(d) acyler un composé de formule I dans laquelle $R^1$ représente $NH_2$ pour obtenir un composé de formule I dans laquelle $R^1$ représente $NHR^3$ où $R^3$ représente $R^6$-CO ; et
(e) éventuellement faire suivre d'une salification pour obtenir le sel d'addition d'acide pharmaceutiquement acceptables du produit de formule I et/ou du dédoublement d'un produit racémique pour obtenir le produit optiquement actif de formule I.

2. Procédé selon la revendication 1, pour préparer le stéréo-isomère trans-(-) de formule I.

3. Procédé selon la revendication 1 ou 2, pour préparer un composé de formule I, dans laquelle R représente un groupe n-propyle, $R^1$ représente un groupe amino et $R^2$ représente un atome d'hydrogène.

4. Procédé selon la revendication 1, pour préparer la trans-(±)-2-amino-6-méthyl-5,5a,6,7,8,9,9a,10-octahydropyrimido[4,5-g]quinoléine, ce procédé consistant à faire réagir la trans-(±)-1-méthyl-6-oxo-7-(diméthylaminométhylène)-décahydroquinoléine avec du carbonate de guanidine.

5. Procédé selon la revendication 1, pour préparer la trans-(±)-2-amino-6-n-propyl-5,5a,6,7,8,9,9a,10-octahydropyrimido[4,5-g]quinoléine, ce procédé consistant à faire réagir la trans-(±)-1-n-propyl-6-oxo-7-(diméthylaminométhylène)-décahydroquinoléine avec du carbonate de guanidine.

6. Procédé selon la revendication 2, pour préparer la 5aR,9aR-2-amino-6-n-propyl-5,5a,6,7,8,9,9a,10-octahydropyrimido[4,5-g]quinoléine, ce procédé consistant à faire réagir la 4aR,8aR-1-n-propyl-6-oxo-7-(diméthylaminométhylène)-décahydroquinoléine avec du carbonate de guanidine.

7. Procédé selon la revendication 1, pour préparer la trans-(±)-2-diméthylamino-6-n-propyl-5,5a,6,7,8,9,9a,10-octahydropyrimido[4,5-g]quinoléine, ce procédé consistant à faire réagir la trans-(±)-1-n-propyl-6-oxo-7-(diméthylaminométhylène)-décahydroquinoléine avec du chlorhydrate de N,N-diméthylguanidine.

8. Procédé selon la revendication 1, pour préparer la trans-(±)-2-méthylamino-6-n-propyl-5,5a,6,7,8,9,9a,10-octahydropyrimido[4,5-g]quinoléine, ce procédé consistant à faire réagir la trans-(±)-1-n-propyl-6-oxo-7-(diméthylaminométhylène)-décahydroquinoléine avec de la N-méthylguanidine.

9. Procédé selon la revendication 1, pour préparer la trans-(±)-2-amino-4-méthyl-6-n-propyl-5,5a,6,7,8,9,9a,10-octahydropyrimido[4,5-g]quinoléine, ce procédé consistant à faire réagir la trans-(±)-1-n-propyl-6-oxo-7-acétyldécahydroquinoléine avec du carbonate de guanidine.

10. Procédé selon la revendication 1, pour préparer la trans-(±)-2-amino-4-chloro-6-n-propyl-5,5a,6,7,8,9,9a,10-octahydropyrimido[4,5-g]quinoléine, ce procédé consistant à faire réagir la trans-(±)-2-amino-4-hydroxy-6-n-propyl-5,5a,6,7,8,9,9a,10-octahydropyrimido[4,5-g]quinoléineavec de l'oxychlorure phosphoreux.

11. Procédé selon la revendication 1, pour préparer la trans-(±)-2-acétylamino-6-n-propyl-5,5a,6,7,8,9,9a,10-octahydropyrimido[4,5-g]quinoléine, ce procédé consistant à faire réagir la trans-(±)-2-amino-6-n-propyl-5,5a,6,7,8,9,9a,10-octahydropyrimido[4,5-g]quinoléine avec de l'anhydride acétique.

12. Procédé selon la revendication 1, pour préparer la trans-(±)-2-benzoylamino-6-n-propyl-5,5a,6,7,8,9,9a,10-octahydropyrimido[4,5-g]quinoléine, ce procédé consistant à faire réagir la trans-(±)-2-amino-6-n-propyl-5,5a,6,7,8,9,9a,10-octahydropyrimido[4,5-g]quinoléine avec du chlorure de benzoyle.

13. Procédé selon la revendication 1, pour préparer la 5aS,9aS-2-amino-6-n-propyl-5,5a,6,7,8,9,9a,10-octahydropyrimido[4,5-g]quinoléine, ce procédé consistant à faire réagir la 4aS,8aS-1-n-propyl-6-oxo-7-(diméthylaminométhylène)-décahydroquinoléine avec du carbonate de guanidine.

14. Procédé selon la revendication 1 pour préparer la trans-(-)-2-amino-6-n-propyl-5,5a,6,7,8,9,9a,10-octahydropyrimidoquinoléine ou un de ses sels d'addition d'acides pharmaceutiquement acceptables.

15. Procédé selon la revendication 1 pour préparer le dichlorhydrate de la trans-(-)-2-amino-6-n-propyl-5,5a,6,7,8,9,9a,10-octahydropyrimidoquinoléine.

## Ansprüche

1. Verbindung der Formel

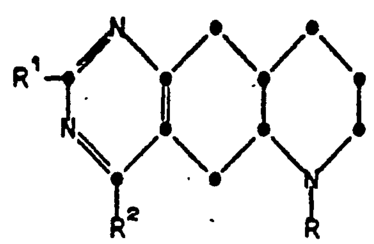

I

worin
R ein $C_1$-$C_3$-Alkyl- oder Allylrest ist;
$R^2$ H, $CH_3$, Cl oder Br ist;
$R^1$ $NH_2$, $NHR^3$ oder $NR^4R^5$ ist;
worin $R^3$ ein Methyl-, Ethyl-, n-Propylrest oder $R^6$-CO ist, wobei $R^6$ ein $C_1$-$C_3$-Alkylrest oder

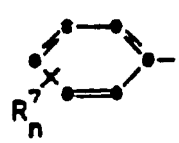

ist, worin $R^7$ unabhängig H, Cl, F, Br, $CH_3$, $C_2H_5$, $CH_3O$, $C_2H_5O$ oder $CF_3$ ist und n 0, 1 oder 2 ist; worin $R^4$ und $R^5$ unabhängig Methyl-, Ethyl- oder n-Propylreste sind; oder ein pharmazeutisch annehmbares Säureadditionssalz davon.

2. Verbindung nach Anspruch 1, die das trans-(-)-Stereoisomer der Formel

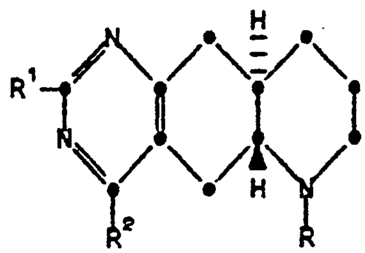

II

ist, worin R, $R^1$ und $R^2$ wie in Anspruch 1 definiert sind, oder ein pharmazeutisch annehmbares Säureadditionssalz davon.

3. Verbindung nach Anspruch 1, die das trans-(+)-Stereoisomer der Formel

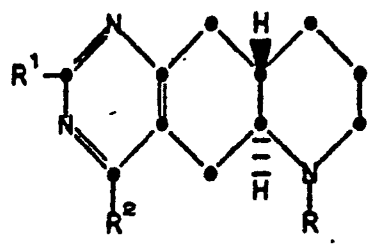

IIa

ist, worin R, $R^1$ und $R^2$ wie in Anspruch 1 definiert sind, oder ein pharmazeutisch annehmbares Säureadditionssalz davon.

4. Verbindung nach Anspruch 1, 2 oder 3 der Formel I, II oder IIa, worin R ein n-Propylrest ist.

5. Eine der folgenden Verbindungen oder ein pharmazeutisch annehmbares Säureadditionssalz davon:
trans-(±)-2-Amino-6-methyl-5,5a,6,7,8,9,9a,10-octahydropyrimido[4,5-g]chinolin,
trans-(±)-2-Amino-6-n-propyl-5,5a,6,7,8,9,9a,10-octahydropyrimido[4,5-g]chinolin,
5aR,9aR-2-Amino-6-n-propyl-5,5a,6,7,8,9,9a,10-octahydropyrimido[4,5-g]chinolin,
trans-(±)-2-Dimethylamino-6-n-propyl-5,5a,6,7,8,9,9a,10-octahydropyrimido[4,5-g]chinolin,
trans-(±)-2-Methylamino-6-n-propyl-5,5a,6,7,8,9,9a,10-octahydropyrimido[4,5-g]chinolin,
trans-(±)-2-Amino-4-methyl-6-n-propyl-5,5a,6,7,8,9,9a,10-octahydropyrimido[4,5-g]chinolin,
trans-(±)-2-Amino-4-chlor-6-n-propyl-5,5a,6,7,8,9,9a,10-octahydropyrimido[4,5-g]chinolin,
trans-(±)-2-Acetylamino-6-n-propyl-5,5a,6,7,8,9,9a,10-octahydropyrimido[4,5-g]chinolin,
trans-(±)-2-Benzoylamino-6-n-propyl-5,5a,6,7,8,9,9a,10-octahydropyrimido[4,5-g]chinolin,
5aS,9aS-2-Amino-6-n-propyl-5,5a,6,7,8,9,9a,10-octahydropyrimido[4,5-g]chinolin.

6. Verfahren zur Herstellung einer Verbindung der Formel I, II oder IIa oder eines pharmazeutisch annehmbaren Säureadditionssalzes davon, nach einem der Ansprüche 1 bis 5, umfassend:
(a) daß man eine Verbindung der Formel

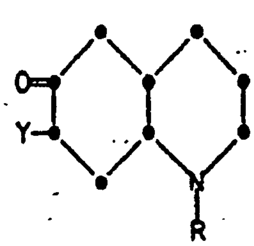

V

worin R wie oben definiert ist;

Y $CH_3CO$ oder $(R^{10})_2NCH=$ ist, worin jeder Rest $R^{10}$ unabhängig ein $C_1$-$C_3$-Alkylrest ist oder ein Rest $R^{10}$ H ist und der andere Rest $R^{10}$ ein $C_1$-$C_3$-Alkylrest ist;
mit einer Verbindung oder einem Salz der Formel

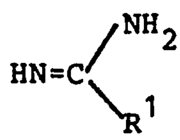

VI

worin $R^1$ wie oben definiert ist, kondensiert, was eine Verbindung der Formel I liefert; oder
(b) eine Verbindung der Formel

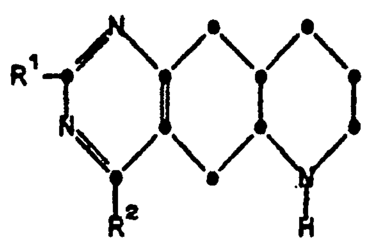

Ia

worin $R^1$ und $R^2$ wie oben definiert sind, alkyliert, was Verbindungen der Formel I liefert; oder
(c) eine Verbindung der Formel

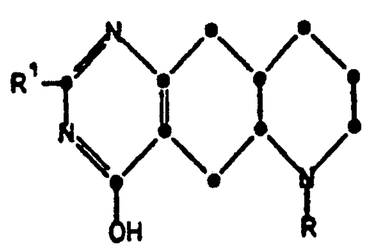

If

worin R und $R^1$ wie oben definiert sind, halogeniert, was die Verbindungen der Formel I liefert, worin $R^2$ Cl oder Br ist; oder
(d) eine Verbindung der Formel I, worin $R^1$ $NH_2$ ist, acyliert, was eine Verbindung der Formel I liefert, worin $R^1$ $NHR^3$ ist, worin $R^3$ $R^6$-CO ist; und
(e) gegebenenfalls anschließend ein Salz bildet, um das pharmazeutisch annehmbare Säureadditionssalz des Produktes der Formel I herzustellen und/oder ein razemisches Produkt auftrennt, um das optisch aktive Produkt der Formel I zu bilden.

**7.** Pharmazeutisches Präparat, umfassend als aktiven Inhaltsstoff eine Verbindung der Formel I, II oder IIa oder ein pharmazeutisch annehmbares Säureadditionssalz davon, nach einem der Ansprüche 1 bis 5 zusammen mit einem oder mehreren pharmazeutischannehmbaren Trägern oder Verdünnungsmitteln dafür.

**8.** Pharmazeutisches Präparat nach Anspruch 7, angepaßt an die orale oder IP-Verabreichung.

**9.** Verbindungen der Formel I oder II oder ein pharmazeutisch annehmbares Säureadditionssalz davon nach Anspruch 1, 2, 4 oder 5 zur Verwendung als D-2-Dopaminagonist.

**10.** Verbindung der Formel I oder IIa oder ein pharmazeutisch annehmbares Säureadditionssalz davon nach Anspruch 1, 3, 4 oder 5 zur Verwendung als D-1-Dopaminagonist.

**11.** Verbindung der Formel

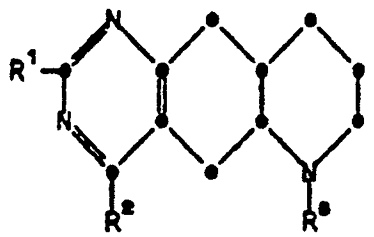

III

worin $R^1$ und $R^2$ wie in Anspruch 1 definiert sind und $R^8$ H, CN oder ($C_1$-$C_3$-Alkoxy)-CO ist, oder ein Säureadditionssalz davon.

**12.** Verbindung nach Anspruch 11, die das trans-(-)-Stereoisomer der Formel

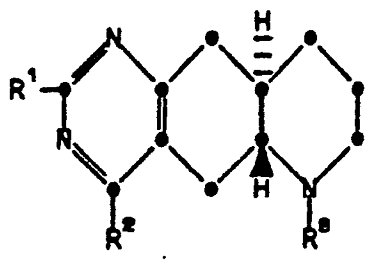

IV

worin $R^1$, $R^2$ und $R^8$ wie in Anspruch 11 definiert sind, ist, oder ein Säureadditionssalz davon.

**13.** Verbindung nach Anspruch 11, die das trans-(+)-Stereoisomer der Formel

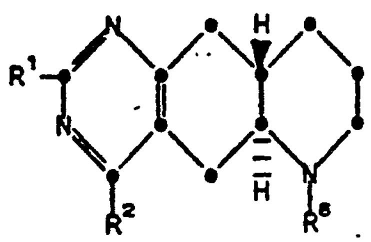

IVa

worin $R^1$, $R^2$ und $R^8$ wie in Anspruch 11 definiert sind, ist, oder ein Säureadditionssalz davon.

**14.** trans-(±)-Verbindung der Formel

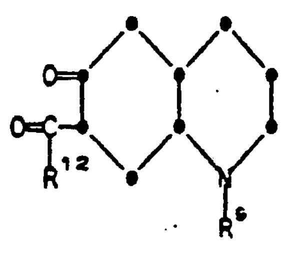

Va

worin $R^9$ ein $C_1$-$C_3$-Alkylrest, CN, H oder ein Allylrest ist, und $R^{12}$ ein Methyl- oder $C_1$-$C_3$-Alkyloxyrest ist, oder ein Säureadditionssalz ist.

**15.** trans-(-)-Stereoisomer einer Verbindung nach Anspruch 14.

**16.** trans-(+)-Stereoisomer einer Verbindung nach Anspruch 14.

**17.** trans-(-)-2-Amino-6-n-propyl-5,5a,6,7,8,9,9a,10-octahydropyrimidochinolin oder ein pharmazeutisch annehmbares Salz davon.

**18.** trans-(-)-2-Amino-6-n-propyl-5,5a,6,7,8,9,9a,10-octahydropyrimidochinolindihydrochlorid.

Patentansprüche für folgenden Vertragsstaat: AT

**1.** Verfahren zur Herstellung einer Verbindung der Formel

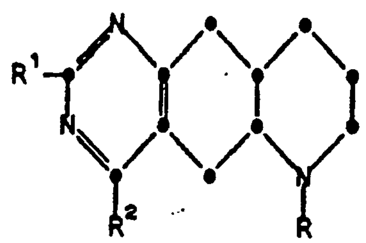

I

worin
R ein $C_1$-$C_3$-Alkyl- oder Allylrest ist;
$R^2$ H, $CH_3$, Cl oder Br ist;
$R^1$ $NH_2$, $NHR^3$ oder $NR^4R^5$ ist;
worin R ein Methyl-, Ethyl-, n-Propylrest oder $R^6$-CO ist, wobei $R^6$ ein $C_1$-$C_3$-Alkylrest oder

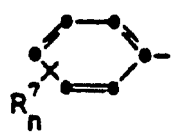

ist, worin $R^7$ unabhängig H, Cl, F, Br, $CH_3$, $C_2H_5$, $CH_3O$, $C_2H_5O$ oder $CF_3$ ist und n 0, 1 oder 2 ist; worin $R^4$ und $R^5$ unabhängig Methyl-, Ethyl- oder n-Propylreste sind; oder eines pharmazeutisch annehmbaren Säureadditionssalzes davon, umfassend

(a) daß man eine Verbindung der Formel

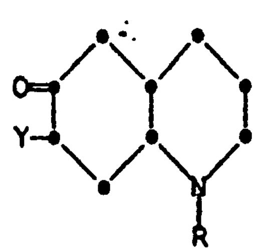

V

worin R wie oben definiert ist;
Y $CH_3CO$ oder $(R^{10})_2NCH=$ ist, worin jeder Rest $R^{10}$ unabhängig ein $C_1$-$C_3$-Alkylrest ist oder ein Rest $R^{10}$ H ist und der andere Rest $R^{10}$ ein $C_1$-$C_3$-Alkylrest ist;
mit einer Verbindung oder einem Salz der Formel

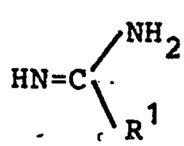

VI

worin $R^1$ wie oben definiert ist, kondensiert, was eine Verbindung der Formel I liefert; oder

(b) eine Verbindung der Formel

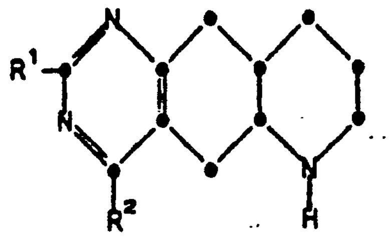

Ia

worin $R^1$ und $R^2$ wie oben definiert sind, alkyliert, was Verbindungen der Formel I liefert; oder
(c) eine Verbindung der Formel

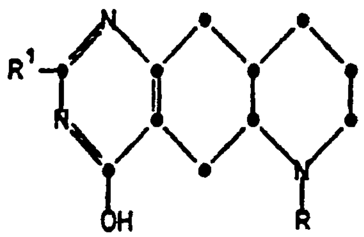

If

worin R und $R^1$ wie oben definiert sind, halogeniert, was die Verbindungen der Formel I liefert, worin $R^2$ Cl oder Br ist; oder
(d) eine Verbindung der Formel I, worin $R^1$ $NH_2$ ist, acyliert, was eine Verbindung der Formel I liefert, worin $R^1$ $NHR^3$ ist, worin $R^3$ $R^6$-CO ist; und
(e) gegebenenfalls anschließend ein Salz bildet, um das pharmazeutisch annehmbare Säureadditionssalz des Produktes der Formel I herzustellen und/oder ein razemisches Produkt auftrennt, um das optisch aktive Produkt der Formel I zu bilden.

**2.** Verfahren nach Anspruch 1 zur Herstellung des trans-(-)-Stereoisomers von Formel I.

**3.** Verfahren nach Anspruch 1 oder 2 zur Herstellung einer Verbindung der Formel I, worin R ein n-Propylrest ist, $R^1$ ein Aminorest ist und $R^2$ Wasserstoff ist.

**4.** Verfahren nach Anspruch 1 zur Herstellung von trans-(±)-2-Amino-6-methyl-5,5a,6,7,8,9,9a,10-octahydropyrimido[4,5-g]chinolin, umfassend, daß man trans-(±)-1-Methyl-6-oxo-7-[dimethylaminomethylen)-decahydrochinolin mit Guanidincarbonat umsetzt.

**5.** Verfahren nach Anspruch 1 zur Herstellung von trans-(±)-2-Amino-6-n-propyl-5,5a,6,7,8,9,9a,10-octahydropyrimido[4,5-g]chinolin,umfassend, daß man trans-(±)-1-n-Propyl-6-oxo-7-(dimethylaminomethylen)-decahydrochinolin mit Guanidincarbonat umsetzt.

**6.** Verfahren nach Anspruch 2 zur Herstellung von 5aR,9aR-2-Amino-6-n-propyl-5,5a,6,7,8,9,9a,10-octahydropyrimido[4,5-g]chinolin, umfassend, daß man 4aR,8aR-1-n-Propyl-6-oxo-7-(dimethylaminomethylen)-decahydrochinolin mit Guanidincarbonat umsetzt.

**7.** Verfahren nach Anspruch 1 zur Herstellung von trans-(±)-2-Dimethylamino-6-n-propyl-5,5a,6,7,8,9,9a,10-octahydropyrimido[4,5-g]chinolin, umfassend, daß man trans-(±)-1-n-Propyl-6-oxo-7-(dimethylaminomethylen)-decahydrochinolin mit N,N-Dimethylguanidinhydrochlorid umsetzt.

**8.** Verfahren nach Anspruch 1 zur Herstellung von trans-(±)-2-Methylamino-6-n-propyl-5,5a,6,7,8,9,9a,10-octahydropyrimido[4,5-g]chinolin, umfassend, daß man trans-(±)-1-n-Propyl-6-oxo-7-(dimethylaminomethylen)-decahydrochinolin mit N-Methylguanidin umsetzt.

**9.** Verfahren nach Anspruch 1 zur Herstellung von trans-(±)-2-Amino-4-methyl-6-n-propyl-5,5a,6,7,8,9,9a,10-octahydropyrimido[4,5-g]chinolin, umfassend, daß man trans-(±)-1-n-Propyl-6-oxo-7-acetyldecahydrochinolin mit Guanidincarbonat umsetzt.

10. Verfahren nach Anspruch 1 zur Herstellung von trans-(±)-2-Amino-4-chlor-6-n-propyl-5,5a,6,7,8,9,9a,10-octahydropyrimido[4,5-g]chinolin, umfassend, daß man trans-(±)-2-Amino-4-hydroxy-6-n-propyl-5,5a,6,7,8,9,9a,10-octahydropyrimido[4,5-g]chinolin mit Phosphoroxychlorid umsetzt.

11. Verfahren nach Anspruch 1 zur Herstellung von trans-(±)-2-Acetylamino-6-n-propyl-5,5a,6,7,8,9,9a,10-octahydropyrimido[4,5-g]chinolin, umfassend, daß man trans-(±)-2-Amino-6-n-propyl-5,5a,6,7,8,9,9a,10-octahydropyrimido[4,5-g]chinolin mit Essigsäureanhydrid umsetzt.

12. Verfahren nach Anspruch 1 zur Herstellung von trans-(±)-2-Benzoylamino-6-n-propyl-5,5a,6,7,8,9,9a,10-octahydropyrimido[4,5-g]chinolin,umfassend, daß man trans-(±)-2-Amino-6-n-propyl-5,5a,6,7,8,9,9a,10-octahydropyrimido[4,5-g]chinolin mit Benzoylchlorid umsetzt.

13. Verfahren nach Anspruch 1 zur Herstellung von 5aS,9aS-2-Amino-6-n-propyl-5,5a,6,7,8,9,9a,10-octahydropyrimido[4,5-g]chinolin, umfassend, daß man 4aS,8aS-1-n-Propyl-6-oxo-7-(dimethylaminomethylen)-decahydrochinolin mit Guanidincarbonat umsetzt.

14. Verfahren nach Anspruch 1 zur Herstellung von trans-(-)-2-Amino-6-n-propyl-5,5a,6,7,8,9,9a,10-octahydropyrimidochinolin oder eines pharmazeutisch annehmbaren Salzes davon.

15. Verfahren nach Anspruch 1 zur Herstellung von trans-(-)-2-Amino-6-n-propyl-5,5a,6,7,8,9,9a,10-octahydropyrimidochinolindihydrochlorid.